(19) Europäisches Patentamt
European Patent Office
Office européen des brevets

(11) Publication number: **0 505 954 A1**

# (12) EUROPEAN PATENT APPLICATION

(21) Application number: **92104951.6**

(22) Date of filing: **21.03.92**

(51) Int. Cl.[5]: **C07K 5/06**, C07D 405/14, C07D 405/06, A61K 31/41

(30) Priority: **25.03.91 GB 9106350**
**16.05.91 GB 9110619**

(43) Date of publication of application:
**30.09.92 Bulletin 92/40**

(84) Designated Contracting States:
**AT BE CH DE DK ES FR GB GR IT LI LU MC NL PT SE**

(71) Applicant: **GLAXO GROUP LIMITED**
**Glaxo House, Berkeley Avenue**
**Greenford, Middlesex UB6 0NN(GB)**

(72) Inventor: **Hirst, Gavin Charles, Glaxo Inc.**
**Five Moore Drive, Box 13398**
**Res. Triangle Park, North Carolina 27709(US)**
Inventor: **Ross, Barry Clive**
**Glaxo Group Res.Ltd., Park Road**
**Ware, Hertfordshire, SG12 0DG(GB)**
Inventor: **Middlemiss, David**
**Glaxo Group Res.Ltd., Park Road**
**Ware, Hertfordshire, SG12 0DG(GB)**
Inventor: **Scopes, David Ian Carter**
**Glaxo Group Res.Ltd., Park Road**
**Ware, Hertfordshire, SG12 0DG(GB)**
Inventor: **Jack, Torquil Iain MacLean**
**Glaxo Group Res.Ltd., Park Road**
**Ware, Hertfordshire, SG12 0DG(GB)**
Inventor: **Cardwell, Kevin Stuart**
**Glaxo Group Res.Ltd., Park Road**
**Ware, Hertfordshire, SG12 0DG(GB)**
Inventor: **Dowle, Michael Dennis**
**Glaxo Group Res.Ltd., Park Road**
**Ware, Hertfordshire, SG12 0DG(GB)**
Inventor: **Watson, Stephen Paul**
**Glaxo Group Res.Ltd., Park Road**
**Ware, Hertfordshire, SG12 0DG(GB)**

(74) Representative: **James, Stephen Richard, Dr. et al**
**Glaxo Holdings plc Glaxo House Berkeley Avenue**
**Greenford, Middlesex UB6 0NN(GB)**

## (54) N-Imidazolylmethyl benzofuran derivatives as inhibitors of angiotensin II activity.

(57) The invention provides compounds of the general formula (I):

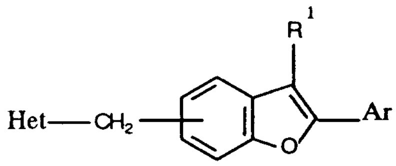

(I)

or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy ,-CHO, $-CO_2H$ or $-COR^2$;
Ar represents the group

EP 0 505 954 A1

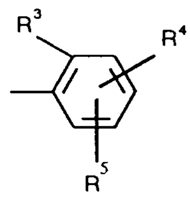 ;

$R^2$ represents a group selected from $C_{1-6}$alkyl, $C_{2-6}$alkenyl, $C_{1-6}$alkoxy or the group $-NR^{12}R^{13}$;
$R^3$ represents a group selected from $-CO_2H$, $-NHSO_2CF_3$ or a C-linked tetrazolyl group;
$R^4$ and $R^5$ which may be the same or different each independently represent a hydrogen atom or a halogen atom or a $C_{1-6}$alkyl group;
Het represents the group

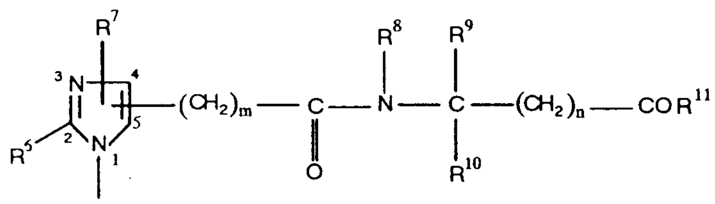 ;

$R^6$ represents a hydrogen atom or a group selected from $C_{1-6}$alkyl, $C_{2-6}$alkenyl, $C_{1-6}$alkylthio, $C_{1-6}$alkoxy, $C_{3-7}$cycloalkyl or $C_{3-7}$cycloalkyl$C_{1-4}$alkyl;
$R^7$ represents a hydrogen atom or a halogen atom or a group selected from $C_{1-6}$alkyl, $C_{2-6}$alkenyl or fluoro$C_{1-6}$alkyl;
$R^8$ represents a hydrogen atom or a $C_{1-6}$alkyl group;
$R^9$ represents a hydrogen atom or a group selected from phenyl or $C_{1-6}$alkyl, optionally substituted by a phenyl, hydroxyphenyl, hydroxyl, amino, guanidino, imidazolyl, indolyl, mercapto, $C_{1-6}$alkylthio, carboxyl or amido group; or
$R^8$ and $R^9$, when taken together, form an alkylene bridge of 2, 3 or 4 carbon atoms;
$R^{10}$ represents a hydrogen atom or a $C_{1-6}$alkyl group;
$R^{11}$ represents a group selected from hydroxyl, $C_{1-6}$alkoxy, $C_{2-6}$alkenoxy or $-NR^{12}R^{13}$;
$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents zero or an integer from 1 to 4; and
n represents zero or an integer from 1 to 3; with the proviso that when n represents an integer from 1 to 3, $R^9$ and $R^{10}$ both represent hydrogen atoms.

The compounds may be used in the treatment or prophylaxis of hypertension and diseases associated with cognitive disorders.

This invention relates to benzofuran derivatives, processes for their preparation and pharmaceutical compositions containing them. According to a first aspect of the invention we provide a compound of the general formula (I):

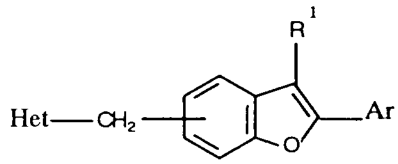

(I)

or a physiologically acceptable salt, solvate (e.g. hydrate) or metabolically labile ester thereof in which
$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy ,-CHO, $-CO_2H$ or $-COR^2$;
Ar represents the group

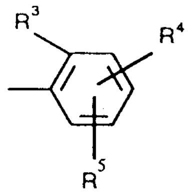

;

$R^2$ represents a group selected from $C_{1-6}$alkyl, $C_{2-6}$alkenyl, $C_{1-6}$alkoxy or the group $-NR^{12}R^{13}$;
$R^3$ represents a group selected from $-CO_2H$, $-NHSO_2CF_3$ or a C-linked tetrazolyl group;
$R^4$ and $R^5$ each independently represent a hydrogen atom or a halogen atom or a $C_{1-6}$alkyl group;
Het represents the group

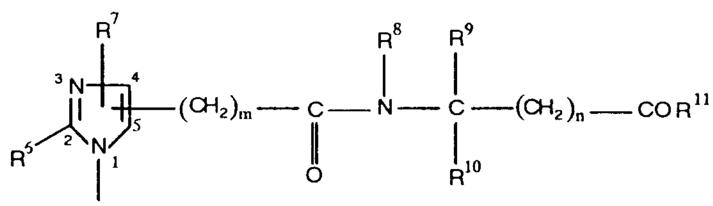

;

$R^6$ represents a hydrogen atom or a group selected from $C_{1-6}$alkyl, $C_{2-6}$alkenyl, $C_{1-6}$alkylthio, $C_{1-6}$alkoxy, $C_{3-7}$cycloalkyl or $C_{3-7}$cycloalkyl$C_{1-4}$alkyl;
$R^7$ represents a hydrogen atom or a halogen atom or a group selected from $C_{1-6}$alkyl, $C_{2-6}$alkenyl or fluoro$C_{1-6}$alkyl;
$R^8$ represents a hydrogen atom or a $C_{1-6}$alkyl group;
$R^9$ represents a hydrogen atom or a group selected from phenyl or $C_{1-6}$alkyl, optionally substituted by a phenyl, hydroxyphenyl, hydroxyl, amino, guanidino, imidazolyl, indolyl, mercapto, $C_{1-6}$alkylthio, carboxyl or amido group; or
$R^8$ and $R^9$, when taken together, form an alkylene bridge of 2, 3 or 4 carbon atoms;
$R^{10}$ represents a hydrogen atom or a $C_{1-6}$alkyl group;
$R^{11}$ represents a group selected from hydroxyl, $C_{1-6}$alkoxy, $C_{2-6}$alkenoxy or $-NR^{12}R^{13}$;
$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents zero or an integer from 1 to 4; and
n represents zero or an integer from 1 to 3; with the proviso that when n represents an integer from 1 to 3, $R^9$ and $R^{10}$ both represent hydrogen atoms.

Where optical isomers may exist formula (I) is intended to cover all enantiomers, diastereoisomers and mixtures thereof including racemates. Compounds containing one or two double bonds may exist in the cis or trans configuration.

It should be noted that when $R^9$ and $R^{10}$ are different (and n is therefore zero) in the amino acid moiety

represented thus:

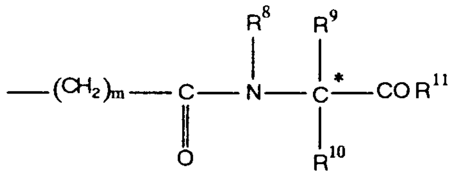

the configuration around the asterisked carbon atom may be in the S or R form.

The invention also includes within its scope the solvates, especially the hydrates of compounds of general formula (I).

Within the above definition the term 'alkyl', 'alkoxy' 'alkylthio' as a group or part of a group means that the group is straight or branched. The term 'alkenyl' as a group or part of a group means that the group is straight or branched and contains at least one carbon-carbon double bond. The term 'cycloalkyl' as a group or part of a group may be, for example, a cyclopropyl, cyclobutyl, cyclopentyl or cyclohexyl group.

The term 'halogen' means a fluorine, chlorine, bromine or iodine atom.

The term 'fluoro$C_{1-6}$alkyl' means a $C_{1-6}$alkyl group in which one or more hydrogen atoms have been replaced by a fluorine atom, for example, $-CH_2CF_3$.

Within the above definition when $-NR^{12}R^{13}$ represents a saturated heterocyclic ring, this contains 5 or 6 ring members, one of which may be an oxygen atom. Suitable heterocyclic groups are a pyrrolidino, piperidino or morpholino group.

The group $R^9$ may be, for example, a hydrogen atom or a group selected from $C_{1-4}$alkyl (e.g. methyl, isopropyl, s-butyl, isobutyl), hydroxymethyl, 1-hydroxyethyl, p-hydroxybenzyl, benzyl, 3-indolylmethyl, $-CH_2CO_2H$, $-(CH_2)_2CO_2H$, $-(CH_2)_4NH_2$, $-(CH_2)_3NHC(=NH)NH_2$, 3-imidazolylmethyl, $-CH_2SH$, $-(CH_2)_2SCH_3$, $-CH_2CONH_2$ or $-(CH_2)_2CONH_2$.

Where $R^8$ and $R^9$ are joined through the nitrogen and carbon atoms to which they are attached to form a 4 to 6 membered ring the group Het may be, for example,

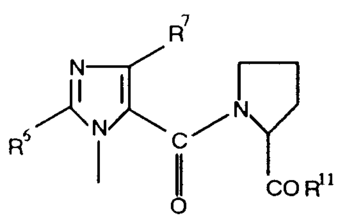

A particularly preferred class of compounds of general formula (I) is that wherein $R^9$ represents a hydrogen atom or a $C_{1-4}$alkyl group optionally substituted by hydroxyl, phenyl, $-CO_2H$, amino or $-CONH_2$, or where $R^8$ and $R^9$ together form a propylene bridge. In particular, $R^9$ represents a hydrogen atom or a $C_{1-4}$alkyl group (e.g. a methyl group).

A further preferred class of compounds of general formula (I) is that wherein $R^6$ represents a hydrogen atom or a $C_{1-5}$alkyl, or a $C_{3-5}$alkenyl group. Particularly preferred are those compounds wherein the 2-position substituent is a $C_{2-4}$alkyl group (especially an ethyl, n-propyl or n-butyl group).

Another preferred class of compounds of general formula (I) is that wherein $R^6$ represents a $C_{3-5}$cycloalkyl or $C_{3-5}$cycloalkyl$C_{1-2}$alkyl group, in particular a cyclopropyl, cyclobutyl or cyclopropylmethyl group.

A yet further preferred class of compounds of general formula (I) is that wherein $R^1$ represents a hydrogen atom or a halogen atom or a group selected from $C_{1-6}$alkyl, $C_{1-6}$alkoxy or fluoro$C_{1-6}$alkyl, and in particular a halogen atom, especially bromine.

Another preferred class of compounds of general formula (I) is that wherein m represents zero or 1, and especially zero. Also preferred are those compounds of general formula (I) wherein n represents zero or 1, and especially zero.

A further preferred class of compounds of general formula (I) is that wherein $R^7$ represents a halogen atom (especially chlorine) or a $C_{1-4}$alkyl group (especially methyl). Also preferred are those compounds of general formula (I) wherein $R^7$ is attached to the imidazole ring at the 4-position.

Another preferred class of compounds of general formula (I) is that wherein $R^8$ represents a hydrogen

atom. Also preferred are those compounds of general formula (I) wherein $R^{10}$ represents a hydrogen atom.

A particularly preferred class of compounds of general formula (I) is that wherein $R^{11}$ represents a hydroxyl group or a $C_{1-4}$alkoxy group (especially a methoxy, ethoxy or propoxy group). Also preferred are those compounds wherein $R^{11}$ represents the group $-NR^{12}R^{13}$, especially where $R^{12}$ and $R^{13}$ each represent a hydrogen atom.

Conveniently, in the compounds of general formula (I), the group Het-$CH_2$- is attached at the 5- or 6-position on the benzofuran ring, and especially the 5-position.

Also conveniently, in the compounds of general formula (I), $R^3$ may be a C-linked tetrazolyl group.

Still conveniently, in the compounds of general formula (I), $R^4$ and $R^5$ may each independently represent a hydrogen atom or a halogen atom. In particular $R^4$ and $R^5$ each represent hydrogen atoms.

Preferred compounds of the present invention include:

N-[[1-[[3-Bromo-2-[2-(1H-tetrazol-5-yl)phenyl]-5-benzofuranyl]methyl]-4-chloro-2-ethyl-1H-imidazol-5-yl]-carbonyl]glycine methyl ester;

N-[[1-[[3-Bromo-2-[2-(1H-tetrazol-5-yl)phenyl]-5-benzofuranyl]methyl]-4-chloro-2-ethyl-1H-imidazol-5-yl]-carbonyl]glycine ethyl ester;

N-[[1-[[3-Bromo-2-[2-(1H-tetrazol-5-yl)phenyl]-5-benzofuranyl]methyl]-4-chloro-2-ethyl-1H-imidazol-5-yl]-carbonyl]-L-alanine ethyl ester;

N-[[1-[[3-Bromo-2-[2-(1H-tetrazol-5-yl)phenyl]-5-benzofuranyl]methyl]-4-chloro-2-propyl-1H-imidazol-5-yl]-carbonyl]glycine;

N-[[1-[[3-Bromo-2-[2-(1H-tetrazol-5-yl)phenyl]-5-benzofuranyl]methyl]-2-butyl-4-chloro-1H-imidazol-5-yl]-carbonyl]-L-alanine ethyl ester;

N-[[1-[[3-Bromo-2-[2-(1H-tetrazol-5-yl)phenyl]-5-benzofuranyl]methyl]-2-butyl-4-chloro-1H-imidazol-5-yl]-carbonyl]-D-alanine ethyl ester;

N-[[1-[[3-Bromo-2-[2-[[(trifluoromethyl)sulphonyl]amino]phenyl]-5-benzofuranyl]methyl]-2-butyl-4-chloro-1H-imidazol-5-yl]carbonyl] glycine ethyl ester;

2-[3-Bromo-5-[[2-butyl-4-chloro-5-[[(2-hydroxy-2-oxoethyl)amino] carbonyl]-1H-imidazol-1-yl]methyl]-2-benzofuranyl]benzoic acid; N-[[1-[[3-Bromo-2-[2-[[(trifluoromethyl)sulphonyl]amino]phenyl]-5-benzofuranyl]-methyl]-4-chloro-2-propyl-1H-imidazol-5-yl]carbonyl] glycine;

N-[[1-[[3-Bromo-2-[2-[[(trifluoromethyl)sulphonyl]amino]phenyl]-5-benzofuranyl]methyl]-4-chloro-2-ethyl-1H-imidazol-5-yl]carbonyl]glycine;

N-[[1-[[3-Bromo-2-[2-(1H-tetrazol-5-yl)phenyl]-5-benzofuranyl] methyl]-2-butyl-4-chloro-1H-imidazol-5-yl]-carbonyl]-L-isoleucine;

N-[[1-[[3-Bromo-2-[2-(1H-tetrazol-5-yl)phenyl]-5-benzofuranyl] methyl]-2-butyl-4-chloro-1H-imidazol-5-yl]-carbonyl]-L-phenylalanine;

N-[[1-[[3-Bromo-2-[2-(1H - tetrazol-5-yl)phenyl]-5-benzofuranyl] methyl]-4-chloro-2-ethyl-1H-imidazol-5-yl]-carbonyl]glycine;

and physiologically acceptable salts, solvates and metabolically labile esters thereof.

Particularly preferred compounds of the present invention include:

N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycine methyl ester;

N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycine ethyl ester;

N-[[1-[[3-Bromo-2-[2-(1H-tetrazol-5-yl)phenyl]-5-benzofuranyl]methyl]-4-chloro-2-propyl-1H-imidazol-5-yl]-carbonyl]glycine ethyl ester;

N-[[1-[[3-Bromo-2-[2-(1H-tetrazol-5-yl)phenyl]-5-benzofuranyl]methyl]-2-butyl-4-chloro-1H imidazol-5-yl]-carbonyl]glycine;

N-[[1-[[3-Bromo-2-[2-(1H-tetrazol-5-yl)phenyl]-5-benzofuranyl]methyl]-4-chloro-2-propyl-1H-imidazol-5-yl]-carbonyl]-$\beta$-alanine ethyl ester;

N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycinamide;

N-[[1-[[3-Bromo-2-[2-(1H-tetrazol-5-yl)phenyl]-5-benzofuranyl]methyl]-2-butyl-4-chloro-1H imidazol-5-yl]-carbonyl]glycine;

N-[[1-[[3-Bromo-2-[2-[[(trifluoromethyl)sulphonyl]amino]phenyl]-5-benzofuranyl]methyl]-4-chloro-2-propyl-1H-imidazol-5-yl]carbonyl] glycine ethyl ester;

N-[[1-[[3-Bromo-2-[2-(1H-tetrazol-5-yl)phenyl]-5-benzofuranyl] methyl]-4-chloro-2-ethyl-1H-imidazol-5-yl]-carbonyl]-L-alanine;

N-[[1-[[3-Bromo-2-[2-(1H-tetrazol-5-yl)phenyl]-5-benzofuranyl] methyl]-2-ethyl-4-methyl-1H-imidazol-5-yl]-carbonyl]glycine;

and physiologically acceptable salts, solvates and metabolically labile esters threof.

In accordance with the first aspect of the present invention, there is also provided a compound of the general formula (I) above or a physiologically acceptable salt, solvate (e.g. hydrate) or metabolically labile ester thereof in which

$R^1$ represents a halogen atom;

Ar represents the group

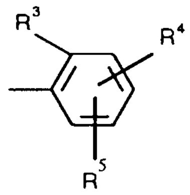

;

$R^3$ represents a group selected from $-CO_2H$, $-NHSO_2CF_3$ or a C-linked tetrazolyl group;

$R^4$ and $R^5$ each independently represent a hydrogen atom;

Het represents the group

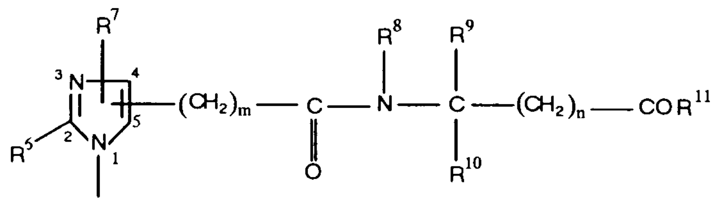

;

$R^6$ represents a $C_{1-6}$alkyl group;

$R^7$ represents a halogen atom or a $C_{1-6}$alkyl group;

$R^8$ represents a hydrogen atom or a $C_{1-6}$alkyl group;

$R^9$ represents a hydrogen atom or a $C_{1-6}$alkyl group, optionally substituted by a phenyl or hydroxyl group, or

$R^8$ and $R^9$, when taken together, form a propylene ($-(CH_2)_3-$) bridge;

$R^{10}$ represents a hydrogen atom;

$R^{11}$ represents a group selected from hydroxyl, $C_{1-6}$alkoxy or $-NH_2$;

m represents zero or an integer from 1 to 4; and

n represents zero or an integer from 1 to 3; with the proviso that when n represents an integer from 1 to 3, $R^9$ and $R^{10}$ both represent hydrogen atoms.

The physiologically acceptable acid addition salts of the compounds of formula (I) may be derived from inorganic or organic acids. Examples of such salts include hydrochlorides, hydrobromides, sulphates, phosphates, benzoates, methanesulphonates or trifluoroacetates.

The compounds may also form salts with suitable bases. Examples of such salts are alkali metal (e.g. sodium or potassium), alkaline earth metal (e.g. calcium or magnesium), ammonium and substituted ammonium (e.g. dimethylammonium, triethylammonium, 2-hydroxyethyldimethylammonium, piperazinium, N,N-dimethylpiperazinium, tetraehtylammonium, piperidinium, ethylenediammonium and choline).

It will be appreciated that, for pharmaceutical use, the salts referred to above will be physiologically acceptable, but other salts may find use, for example, in the preparation of the compounds of general formula (I) and the physiologically acceptable salts thereof.

It will be further appreciated that the compounds of general formula (I) may be chemically modified in the form of compounds which in vivo (for example, by enzymic attack) will provide the parent compounds of general formula (I). Such prodrugs may be, for example, physiologically acceptable metabolically labile ester derivatives. These may be formed by esterification, for example of any of the carboxylic acid groups in the parent compound of general formula (I), with prior protection of any other reactive groups present in the molecule. Examples of such esters include lower alkyl esters (e.g. methyl or ethyl esters), alkenyl esters (e.g. vinyl or alkyl esters), alkynyl esters(e.g. ethynyl or propynyl esters), alkoxyalkyl esters, (e.g. methoxymethyi or 2-methoxyethyl esters), alkylthioalkyl esters (e.g. methylthiomethyl esters) haloalkyl esters (e.g. 2-iodoethyl or 2,2,2-trichloroethyl esters), alkanoyloxyalkyl esters (e.g. acetoxymethyl, 1-acetoxyethyl or pivaloyloxymethyl esters), alkoxycarbonyloxyalkyl esters (e.g. 1-ethoxycarbonyloxyethyl or 1-methoxycarbonyloxyethyl esters), aroyloxyalkyl esters (e.g. benzoyloxymethyl or 1-benzoyloxyethyl esters), substituted or unsubstituted aralkyl esters (e.g. benzyl or 4-amidobenzyl esters), substituted or unsubstituted aminoalkyl esters (e.g aminoethyl or 2-N,N-dimethylaminoethyl esters) or hydroxyalkyl esters (e.g. 2-hydroxyethyl or 2,3-dihydroxypropyl esters).

In addition to the above ester derivatives the present invention includes within its scope compounds of general formula (I) in the form of other physiologically acceptable equivalents, i.e. physiologically acceptable compounds which, like the metabolically labile esters, are converted in vivo into the parent compounds of general formula (I).

According to a second aspect of the present invention we provide a compound of general formula (I) or a physiologically acceptable salt, solvate or metabolically labile ester thereof for use in therapy.

In particular, the compounds of the present invention may be used in the treatment or prophylaxis of hypertension (for example, essential, malignant or resistant, caused by oral contraceptives, coarctation of the aorta or renal vascular disease) and pulmonary hypertension.

The compounds of the present invention may also be used in the treatment or prophylaxis of congestive heart failure, acute or chronic heart failure, aortic or cardiac insufficiency, post-myocardial infarction, renal insufficiency and renal failure (for example, as a result of diabetic nephropathy, glomerular nephritis, scleroderma or renal crisis), proteinuria, Bartter's syndrome, secondary hyperaldosteronism, Reynaud's syndrome, cerebrovascular insufficiency, peripheral vascular disease, diabetic retinopathy, atherogenesis and for the improvement of vascular compliance.

They are also potentially useful for the treatment of cognitive disorders such as dementia (e.g. Alzheimer's disease) and other CNS disorders, such as anxiety disorders, schizophrenia, depression and alcohol or drug (e.g. cocaine) dependency.

According to a further aspect of the present invention we provide a compound of general formula (I) or a physiologically acceptable salt, solvate or metabolically labile ester thereof for use in the treatment of the aforementioned diseases, especially hypertension.

According to another aspect of the present invention we provide a compound of general formula (I) or a physiologically acceptable salt, solvate or metabolically labile ester thereof for the manufacture of a therapeutic agent for the treatment of the aforementioned diseases, especially hypertension.

According to a further aspect of the present invention we provide a method of treating the aforementioned diseases, especially hypertension, which method comprises administering an effective amount to a patient in need of such treatment of a compound of general formula (I) or a physiologically acceptable salt, solvate or metabolically labile ester thereof.

It will be appreciated that the compounds of general formula (I) or a physiologically acceptable salt, solvate, or metabolically labile ester thereof may advantageously be used in conjunction with one or more other therapeutic agents, such as for example diuretics and/or different antihypertensive agents such as $\beta$-blockers, calcium channel blockers or ACE inhibitors. It is to be understood that such combination therapy constitutes a further aspect of the present invention.

It will be further appreciated that reference herein to treatment extends to prophylaxis as well as to the treatment and relief of established symptoms.

While it is possible that a compound of general formula (I) may be administered as the raw chemical it is preferable to present the active ingredient as a pharmaceutical formulation.

The compounds of general formula (I) and their physiologically acceptable salts, solvates and metabolically labile esters may be formulated for administration in any convenient way, and the invention also includes within its scope pharmaceutical compositions comprising at least one compound of general formula (I) or a physiologically acceptable salt, solvate or metabolically labile ester thereof adapted for use in human or veterinary medicine. Such compositions may be presented for use in conventional manner in admixture with one or more physiologically acceptable carriers or excipients. The carrier(s) must be 'acceptable' in the sense of being compatible with the other ingredients of the formulation and not deleterious to the recipient thereof.

Thus, the compounds according to the invention may be formulated for oral, buccal, parenteral or rectal administration or in a form suitable for administration by inhalation or insufflation. Oral administration is preferred.

Tablets and capsules for oral administration may contain conventional excipients such as binding agents, for example mucilage of starch or polyvinylpyrrolidone; fillers, for example, lactose, microcrystalline cellulose or maize-starch; lubricants, for example, magnesium stearate or stearic acid; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups

or elixirs, or may be presented as a dry product for constitution with water or other suitable vehicle before use. Such liquid preparations may contain conventional additives such as suspending agents, for example, sorbitol syrup, methyl cellulose, glucose/sugar syrup or carboxymethyl cellulose; emulsifying agents, for example, sorbitan mono-oleate; non-aqueous vehicles (which may include edible oils), for example, propylene glycol or ethyl alcohol; and preservatives, for example, methyl or propyl p-hydroxybenzoates or sorbic acid. The compounds or their salts or esters may also be formulated as suppositories, e.g. containing conventional suppository bases such as cocoa butter or other glycerides. For buccal administration the composition may take the form of tablets or lozenges formulated in conventional manner.

It will be appreciated that both tablets and capsules may be manufactured in the form of sustained release formulations, such that they provide a controlled continuous release of the compounds according to the invention over a period of hours.

The compounds of general formula (I) and their physiologically acceptable salts, solvates and metabolically labile esters may be formulated for parenteral administration by bolus injection or continuous infusion and may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoro methane, dichlorotetrafluoroethane or other suitable gas. In the case of a pressurised aerosol the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The pharmaceutical formulations according to the invention may also contain other active ingredients such as antimicrobial agents, or preservatives.

It will be appreciated that the amount of a compound of general formula (I) required for use in treatment will vary not only with the particular compound selected but also with the route of administration, the nature of the condition being treated and the age and condition of the patient and will ultimately be at the discretion of the attendant physician or veterinarian. In general, however, when the compositions comprise dosage units, each unit will preferably contain 5mg to 500mg, advantageously where the compounds are to be administered orally 25mg to 400mg of the active compound. The daily dosage as employed for adult human treatment will preferably range from 5mg to 3g, most preferably from 25mg to 1g which may be administered in 1 to 4 daily doses.

The compounds of the invention may be prepared by a number of processes as described below wherein the various groups are as defined for general formula (I) unless otherwise specified.

Thus, according to a further aspect of the present invention we provide a process (A) for preparing the compounds of general formula (I) which comprises treating a compound of general formula (II)

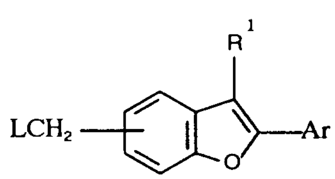

(II)

(wherein L is a leaving group, for example a halogen atom such as chlorine, bromine or iodine, or a hydrocarbylsulphonyloxy group such as methanesulphonyloxy, or p-toluenesulphonyloxy and $R^1$ and Ar are as defined in general formula (I)) with an imidazole of formula (III)

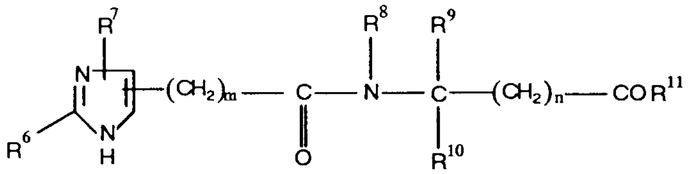

(III)

(wherein $R^6$, $R^7$, $R^8$, $R^9$, $R^{10}$, $R^{11}$, m and n are as defined in general formula (I)) followed by the removal of any protecting groups where present, as described hereinafter.

The reaction is preferably effected under basic conditions, for example, in the presence of sodium hydride, potassium carbonate or sodium methoxide. The reaction is conveniently effected in a solvent such as acetonitrile or an ether e.g. tetrahydrofuran or dioxan, a ketone e.g. butanone or methyl isobutyl ketone, or a substituted amide e.g. dimethylformamide, at a temperature between $0^{0}C$ and the reflux temperature of the solvent.

In another general process (B) a compound of general formula (I) may be obtained by deprotection of a protected intermediate of general formula (IV)

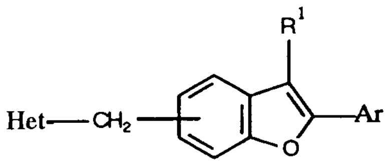

(IV)

(wherein $R^1$, Ar and Het are as defined in general formula (I) except that at least one reactive group is blocked by a protecting group).

The protecting groups may be any conventional protecting groups, for example as described in "Protective Groups in Organic Synthesis" by Theodora Greene (John Wiley and Sons Inc., 1981). Examples of carboxyl protecting groups include $C_{1-6}$alkyl such as methyl or t-butyl, or $C_{7-10}$aralkyl such as benzyl.

When $R^3$ is a tetrazole group, this may be protected with, for example, the trityl group -C(phenyl)$_3$, or a p-nitrobenzyl or 1-ethoxyethyl group.

Deprotection to yield the compound of general formula (I) may be effected using conventional techniques. Thus, for example, aralkyl groups may be cleaved by hydrogenolysis in a suitable organic solvent such as an alcohol, e.g. ethanol, in the presence of a noble metal catalyst such as palladium or an oxide thereof on a support such as charcoal, and conveniently at room temperature and pressure. Carboxyl protecting groups such as alkyl groups may be cleaved by hydrolysis using a base such as an alkali metal hydroxide (e.g. sodium hydroxide or potassium hydroxide) in a suitable solvent (e.g. an aqueous alcohol such as methanol or ethanol) at any suitable temperature up to reflux. Deprotection of the tetrazole group when protected with a trityl group may be effected by acid hydrolysis using trifluoroacetic acid or a mineral acid such as hydrochloric acid in a suitable solvent such as ethanol conveniently at room temperature. Alternatively, when possible, deprotection of the tetrazolyl group can be effected by catalytic hydrogenation as previously described.

In another general process (C) a compound of general formula (I) in which the substituent $R^3$ in the group Ar represents a C-linked tetrazolyl group may be prepared from a compound of general formula (Ia)

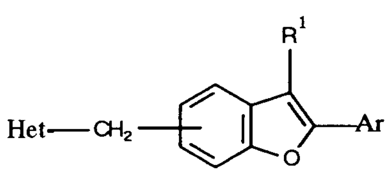

(Ia)

(wherein $R^1$, Ar and Het are as defined in general formula (I) except that in the group Ar, $R^3$ represents a nitrile group) by reaction with a suitable azide such as sodium azide, ammonium azide (preferably prepared in situ from sodium azide and ammonium chloride), trialkyl-(e.g triethyl) ammonium azide (preferably prepared in situ from sodium azide and a trialkylamine (e.g. triethylamine)), a trialkylsilyazide (e.g. trimethylsilylazide) or an alkyl tin azide e.g. tributyl tin azide. The reaction is conveniently effected in a

solvent such as xylene, an ether, for example, dimethoxyethane or tetrahydrofuran, or a substituted amide, for example, dimethylformamide at an elevated temperature, such as the reflux temperature of the solvent, for between 1 and 10 days. Where the azide is tributyl tin azide the reaction may conveniently be effected in the absence of a solvent at a temperature between room temperature and 180°C. Such a reaction leaves the tetrazolyl group protected with a tributyl tin group, which can readily be removed using aqueous base or acid. Where aqueous base is used to effect this deprotection, the compound may be treated with an aqueous acid to liberate the free tetrazole.

Compounds of general formula (Ia) may be prepared by processes analogous to those described herein commencing from a compound of formula (IX) and a corresponding benzofuran intermediate.

The intermediate compounds of general formula (Ia) and their acid addition salts are novel compounds and form a further aspect of the present invention.

In another general process (D) a compound of general formula (I) in which the substituent $R^3$ in the group Ar represents $-NHSO_2CF_3$, may also be prepared from a compound of general formula (Ib)

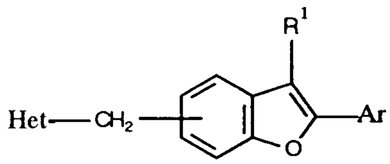

(Ib)

(wherein $R^1$, Ar and Het are as defined in general formula (I) except that in the group Ar, $R^3$ represents an amino group) by reaction with trifluoromethanesulphonic anhydride or trifluoromethylsulphonyl chloride, in a suitable solvent such as a halogenated hydrocarbon, for example, chloroform or dichloromethane in the presence of a base, e.g. triethylamine.

Compounds of general formula (Ib) may be prepared by processes analogous to those described herein commencing from a compound of formula (X) and a corresponding benzofuran intermediate.

Alternatively, compounds of general formula (Ib) may be prepared by a Curtius rearrangement of a compound of formula (I) wherein $R^3$ in the group Ar is $-CO_2H$ (provided that this is the only carboxyl group in the molecule) using, for example, diphenylphosphorylazide in the presence of a base such as triethylamine and in a solvent such as an alcohol (e.g. tert-butanol) to form a carbamate followed by deprotection of the amine in a conventional manner, for example by acid hydrolysis using hydrochloric acid in a solvent such as ethanol.

The intermediate compounds of general formula (Ib) and their acid addition salts are novel compounds and form a further aspect of the present invention.

In another general process (E) a compound of general formula (I) may be obtained by treating a compound of formula (Ic)

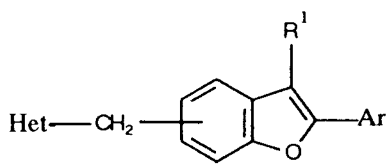

(Ic)

(wherein $R^1$ and Ar are as defined in general formula (I) and Het represents the group

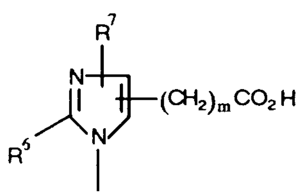

wherein $R^6$, $R^7$ and m are as defined in general formula (I)) with a compound of general formula (V)

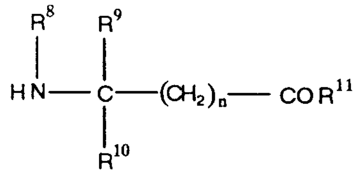

(V)

(wherein $R^8$, $R^9$, $R^{10}$, $R^{11}$ and n are as defined in general formula (I)) followed by the removal of any protecting groups where present, as described above.

The reaction is effected under standard conditions of amide formation, preferably in the presence of a suitable dehydrating agent, such as N,N'-carbonyldiimidazole (CDI) or dicyclohexylcarbodiimide. The reaction is conveniently effected in a solvent such as a substituted amide e.g. dimethylformamide or a halogenated hydrocarbon e.g. dichloromethane or an ether (e.g. tetrahydrofuran) at a temperature between 0° and 100°C, and conveniently at room temperature. See, for example, "Peptide Synthesis, M. Badanski and A.Ondetti, Interscience.

In the processes (A),(B), (C), (D) and (E) described above, the compounds of general formula (I) may be obtained in the form of a salt, conveniently in the form of a physiologically acceptable salt. Where desired, such salts may be converted into the corresponding free acids or free bases using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or isopropanol.

Physiologically acceptable salts may also be prepared from other salts, including other physiologically acceptable salts, of the compounds of general formula (I), using conventional methods.

The intermediate compounds of general formula (II) may be prepared from a compound of formula (VI):

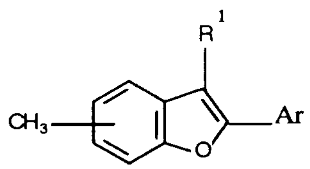

(VI)

using any suitable reagent well known in the art for converting the methyl on the 6-membered ring into the group $-CN_2L$ (wherein L is as defined above). Thus, for example, when L is a halogen atom, a compound of formula (VI) can be converted into a compound of general formula (II) using N-chloro amides, tert-butyl hypochlorite or N-bromosuccinimide. Halogenation of the side chain may be catalysed by light, thus the reaction can be illuminated with a suitable artificial light source, and preferably in the presence of a free radical initiator such as azobisisobutyronitrile (AIBN) or benzoyl peroxide.

Compounds of formula (VI) wherein $R^1$ is a halogen atom, for example, a bromine atom, may be prepared by halogenation of a compound of formula (VI) wherein $R^1$ represents a hydrogen atom, using for example, bromine, in a suitable solvent such as a halogenated hydrocarbon, e.g. carbon tetrachloride.

Compounds of formula (VI) may be prepared by reaction of a compound of formula (VII)

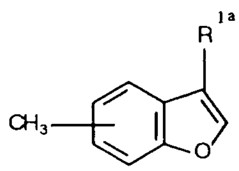

(VII)

(wherein $R^{1a}$ represents a hydrogen atom or a group selected from $C_{1-6}$alkyl or $C_{2-6}$alkenyl) with a compound of formula (VIII)

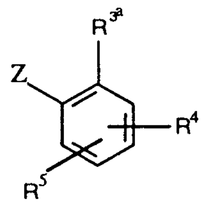

(VIII)

(wherein Z represents a bromine or iodine atom or the group $-OSO_2CF_3$, $R^4$ and $R^5$ are as defined in general formula (I) and $R^{3a}$ is as defined for $R^3$ in general formula (I) or is a protected derivative thereof).

The compound of formula (VII) is first treated with an alkyl lithium compound such as n-butyl lithium at a reduced temperature, for example, between -100°C and 0°C in a solvent such as an ether (e.g. tetrahydrofuran). The mixture is then treated with a trialkylborate such as triisopropylborate and the temperature conveniently brought up to room temperature. Subsequently, water may be added and the mixture treated with a mineral acid such as sulphuric acid thus producing a compound of formula (VIIa)

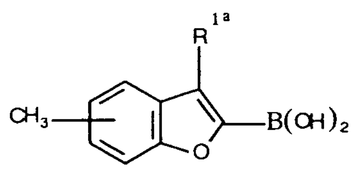

(VIIa)

The intermediate compound of formula (VIIa) is then reacted with a compound of formula (VIII) in the presence of a palladium (0) compound such as tetrakis(triphenylphosphine) palladium (0) in a solvent such as an ether (e.g. dimethoxyethane), and in the presence of a base such as sodium carbonate or thallium hydroxide. The reaction is conveniently effected at an elevated temperature, such as the reflux temperature of the solvent.

Compounds of formula (VI) in which the substituent $R^3$ in the group Ar represents a C-linked tetrazolyl group may be prepared from a precursor of a compound of formula (VI) wherein the substituent $R^3$ represents a nitrile group using the reagents and conditions described in process (C).

Similarly, intermediates of formula (VIII) wherein $R^{3a}$ represents a C-linked tetrazolyl group may be prepared from a compound of formula (IX)

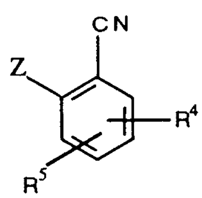

(IX)

(followed where necessary by protection of any reactive groups), using methods well-known in the art such as those described in process (C).

Compounds of formula (VI) in which the substituent $R^3$ in the group Ar is $-NHSO_2CF_3$ may be prepared from a precursor of a compound of formula (VI) wherein the substituent $R^3$ is an amine group using the reagents and conditions described in process (D).

Similarly, intermediates of formula (VIII) wherein $R^{3a}$ represents $-NHSO_2CF_3$ may be prepared from a compound of formula (X),

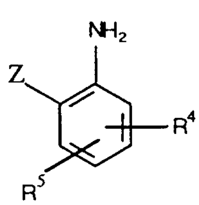

(X)

(followed where necessary by the protection of any reactive group) using methods well known in the art such as those described in process (D).

Compounds of formula (VI) may also be prepared by an intramolecular cyclisation reaction of a compound of formula (XI)

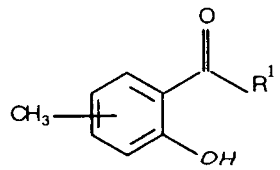

(XI)

(wherein $R^1$ is as previously defined with the exception of CHO and $COR^2$, where $R^2$ is $C_{1-6}$alkoxy or $-NR^{12}R^{13}$) with a suitably substituted benzene of formula (XII)

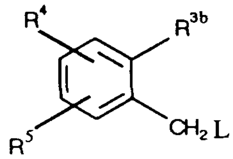

(XII)

(wherein L is as previously defined and $R^{3b}$ is as defined for $R^{3a}$ in formula (VIII) with the exception of $-CO_2H$ and $-NHSO_2CF_3$ or is a nitrile group suitable for subsequent conversion into a tetrazolyl group or is a nitro group suitable for conversion into $-NHSO_2CF_3$),in the presence of a base such as sodium hydride or potassium carbonate. The cyclisation is a two step reaction which requires one equivalent of base per step. It will be appreciated however that the reaction can be effected in the presence of two equivalents of base to avoid the need to isolate the intermediate. The reaction is conveniently effected in a solvent such as an ether e.g tetrahydrofuran, an alcohol e.g ethanol or a substituted amide e.g dimethylformamide, at a temperature between room temperature and the reflux temperature of the solvent.

The intermediates of formula (III) may be prepared from an imidazole of formula (XIII)

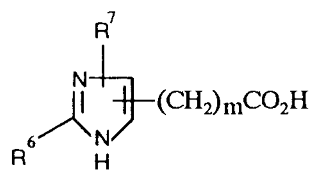

(XIII)

(wherein $R^6$, $R^7$ and m are as defined in general formula (I)) by reaction with a compound of general formula (V) using the reagents and conditions described in process (E).

Compounds of general formula (Ic) may be made by processes analogues to those described herein as (A) to (D) except that the amino acid moiety an ester thereof on the imidazole Het is the group $-(CH_2)_m-CO_2H$ or a group convertible thereto (e.g. an ester thereof or $-(CH_2)_m-CHO$).

It will be appreciated that compounds of formula (VI) in which $R^1$ represents a hydrogen or halogen atom may also be converted into compounds of formula (VI) in which $R^1$ represents the group methyl (via hydrogenolysis of the Mannich base), -CHO or $-COR^2$ (wherein $R^2$ is as defined in general formula (I)) using techniques well known in the art, such as those described in "Heterocyclic Chemistry" by J.A. Joule and G.F. Smith, Van Nostrand Reinhold Company, London (1972), "Heterocyclic Chemistry" by A. Albert, 2nd Edition, The Athlone Press, London (1968), "Heterocyclic Compounds", Vol. 29 by A. Mustafa, John Wiley and Sons Inc., New York (1974), "Heterocyclic Compounds", Vol. 2 by R.C. Elderfield, John Wiley and Sons Inc., New York (1951) and "Advances in Heterocyclic Chemistry", Vol. 29 by A.R. Katritsky and A.J. Boulton, Academic Press, New York (1981).

Intermediates of formulae (VI), (VII), (VIII), (IX), (X), (XI) and (XII), are either known compounds or may be prepared by methods analogous to those used for the preparation of the known compounds.

The following examples illustrate the invention. Temperatures are in $^{0}C$. "Dried" refers to drying using magnesium sulphate. Thin layer chromatography (t.l.c.) was carried out over silica and column chromatography was carried out on silica (Merck 9385 unless otherwise stated), using one of the following solvent

systems : A - ether:hexane, B - ether : petroleum ether, C - dichloromethane:ethanol, D-dichloromethane:ether, or E-ether:acetic acid. The following abbreviations are used : THF - tetrahydrofuran; DME - dimethoxyethane; AIBN - azobisisobutyronitrile; DMF - dimethylformamide; TMEDA - tetramethylethylenediamine; NBS - N-bromosuccinimide; DMAP- 4-dimethylaminopyridine; DEAD - diethyl azodicarboxylate; CDI - carbonyldiimidazole.

Intermediate 1

5-Methylbenzofuran-2-boronic acid

n-Butyl lithium (1.7M, 35.16ml) was added dropwise to a stirred solution of TMEDA (9.58ml) and 5-methylbenzofuran (8.22g) in ether (250ml) maintaining the temperature below - 60°C throughout. The solution was warmed to about -10°C over 45 minutes and stirred at this temperature for 30 minutes. A precipitate formed on warming. The suspension was cooled and triisopropylborate (43ml) was added, maintaining the temperature below -60°C. The solution was warmed gradually to room temperature before quenching with 2N HCl (70ml). The mixture was extracted with ether (3x50ml) and the combined organic extracts washed with 2N HCl (4x30ml), water (2x30ml) and dried before evaporation to give the title compound as an orange solid (12.75g).
t.l.c. System A (1:1), Rf 0.3.

Intermediate 2

2-(5-Methyl-2-benzofuranyl)benzonitrile

Intermediate 1 (20g) was added to a stirred solution of 2-bromobenzonitrile (10.34g) and tetrakistriphenylphosphine palladium (0) (1.5g) in DME (200ml) and 8% $NaHCO_3$ (50ml) at reflux under nitrogen. Further catalyst (1.5g) was added and the reaction continued overnight. The reaction was cooled to room temperature and diluted with ether (200ml). The organic layer was separated, washed with water (3x100ml) and dried. Filtration and evaporation gave a white solid which was purified by chromatography eluting with System A (1:9) to give the title compound (10.58g) as a white solid. T.l.c. System A (1:9), Rf 0.45.
Intermediate 2 was also prepared by the alternative two-step reaction:

a) 2-Hydroxy-5-methylbenzaldehyde

p-Cresol (100g) in dry THF(100ml) was added dropwise to a mechanically stirred, freshly prepared solution of ethyl magnesium bromide [magnesium (25.0g) and bromoethane (75ml)] in THF (500ml) under nitrogen at a rate which maintained a slow reflux (about 30mins). After a further 30mins toluene (1.21) was added, followed by 1,3-dimethyl-3,4,5,6-tetrahydro-2(1H)-pyrimidone (125ml), and paraformaldehyde (70g). The mixture was then heated at reflux for 16h. The mixture was concentrated by distillation and aqueous hydrochloric acid (2M, 600ml) then added. Water (600ml) was added and the mixture filtered through "hyflo". The organic phase was separated, dried, filtered and concentrated in vacuo to give a brown oil. The oil was steam distilled and the product extracted from the distillate with ether (1 litre). The organic extract was dried, filtered and concentrated in vacuo to give a pale yellow slurry which was cooled to -10°C, triturated with ether (precooled to -78°C, 100ml), filtered off rapidly and washed with ether (precooled to -78°C) to give the title compound as colourless needles, (131.4g).
T.l.c. System A (1:5) Rf 0.5.

b) 2-(5-Methyl-2-benzofuranyl)benzonitrile

A solution of the product of step (a) (130g) in dry DMF (400ml) was added dropwise to a solution of sodium methoxide (56.2g) in ethanol (400ml) mechanically stirred under nitrogen. After a further 20mins, a solution of 2-(bromomethyl)benzonitrile (182.2g) in dry DMF (400ml) was added dropwise. The mixture was then heated to 75°C for 30min. The solution was allowed to cool for 1h. A slurry of sodium methoxide (56.2g) in dry DMF (100ml) was added and the mixture heated at reflux for 1.5h. The mixture was concentrated in vacuo and then poured into iced water. The solid was collected, and triturated with methanol to give the title compound (Intermediate 2) as a beige solid (149.4g).
T.l.c. System A (1:9) Rf 0.4.

Intermediate 3

5-[2-(5-Methyl-2-benzofuranyl)phenyl]-1H-tetrazole

A suspension of Intermediate 2 (94g) in tri-n-butyl tin azide (268g) was heated at 100-125$^0$C for 1.25h and at 155-160$^0$C for 2h under nitrogen, then poured into a solution of aqueous sodium hydroxide (0.8N, 3070ml). This solution was then washed with ether and the aqueous phase was acidified to pH1 with 5N hydrochloric acid. The precipitate was filtered, washed with water and dried under vacuum to give the title compound as a buff-coloured solid (100.3g).
T.lc. System A (1:1), Rf 0.2.

Intermediate 4

5-[2-(3-Bromo-5-methyl-2-benzofuranyl)phenyl)-1H-tetrazole

A solution of bromine (58g), in carbon tetrachloride (140ml) was added dropwise over 35min to a mechanically stirred solution of Intermediate 3 (50g) in dry dioxan (2090ml) at room temperature under nitrogen. The resultant solution was stirred at room temperature for 3h, then cyclohexene (63ml) was added. Another preparation of the product was carried out simultaneously on the same scale as described above, and at this stage they were combined. The solvent was evaporated and the residual brown oil (260g) partitioned between ether and aqueous sodium hydroxide. The alkaline solution was acidified to pH1 with hydrochloric acid, then extracted with ethyl acetate. The combined ethyl acetate extracts were washed with brine, dried and evaporated to give a buff solid (125g) which was triturated under hot toluene, cooled and filtered off to give the title compound as a cream coloured solid (101.8g). T.l.c. Ether/petroleum ether/acetic acid (50:50:1), Rf 0.27.

Intermediate 5

5-[2-(3-Bromo-5-methyl-2-benzofuranyl)phenyl]-2-(triphenylmethyl)-2H-tetrazole

Triethylamine (57.4g) was added to a mechanically stirred suspension of Intermediate 4 (101g) in dry dichloromethane (2.9 litres) at room temperature under nitrogen. Triphenylmethyl chloride (79.3g) and DMAP (1.0g) were added at room temperature and the mixture stirred for 3h under nitrogen. The reaction mixture was washed with water, then brine and dried. The solvent was filtered and concentrated to a volume of about 1.2 litres then filtered through silica (Merck 9385, 14cm diam. column). Elution with dichloromethane gave a colourless solid (158.4g) which was triturated with ether and filtered to give the title compound as a colourless solid (147.9g). T.l.c. (Dichloromethane/hexane 1:1), Rf 0.28

Intermediate 6

5-[2-[3-Bromo-5-(bromomethyl)-2-benzofuranyl]phenyl]-2-(triphenylmethyl)-2H-tetrazole

A solution of Intermediate 5 (74g), NBS (22.1g) and benzoyl peroxide (1.1g) in carbon tetrachloride (2050ml) was heated at reflux for 3.25h. under nitrogen. The reaction mixture was cooled and washed with water and brine. Another preparation of the product was carried out simultaneously on the same scale as described above, and at this stage they were combined and dried. The solvent was evaporated to give a colourless solid (168g) which was triturated with ether/methanol (1:1) and filtered to give the title compound as a colourless solid (160.8g).
T.l.c. (Dichloromethane/hexane 1:1), Rf 0.15.

Intermediate 7

2-Butyl-4-chloro-1H-imidazole-5-carboxaldehyde

A suspension of 2-butyl-4-chloro-1H-imidazole-5-methanol (22.0g) in dichloromethane:1,4-dioxan (2:1) (690ml) was treated with manganese dioxide (63.15g) and the reaction mixture heated at reflux under nitrogen for 3.5h. The reaction mixture was cooled, filtered and the filtrate evaporated to leave an off-white solid. The residue was triturated with petroleum ether, filtered and dried to give the title compound as a

white solid (17.9g) m.p. 98-99$^0$C.

Intermediate 8

1-[[3-Bromo-2-[2-[2-(triphenylmethyl)-2H-tetrazol-5-yl]phenyl]-5-benzofuranyl]methyl]-2-butyl-4-chloro-1H-imidazole-5-carboxaldehyde

A mixture of Intermediate 7 (500mg), Intermediate 6 (2.73g) and potassium carbonate (407mg) in dry DMF (20ml) was heated at 80$^0$C for 24h. A further quantity of Intermediate 6 (500mg) was added and heating continued for 18h. The reaction mixture was cooled, poured into water (200ml) and extracted with ethyl acetate. The organic extracts were combined, dried and concentrated in vacuo to a yellow foam (3.6g). This was purified by chromatography, eluting with petroleum ether/ether 3:1, 1:1 and then ether to give the title compound (1.78g) as a pale yellow foam.
T.l.c. System A (1:3) Rf 0.28.

Intermediate 9a

1-[[3-Bromo-2-[2-[2-(triphenylmethyl)-2H-tetrazol-5-yl]phenyl]-5-benzofuranyl]methyl]-2-butyl-4-chloro-1H-imidazole-5-carboxylic acid

A solution of sodium chlorite (3.39g) and sodium dihydrogen phosphate (3.39g) in water (40ml), was added to a mixture of Intermediate 8 (2.93g) in tert-butanol (50ml) and 2-methyl-2-butene (2M in THF, 22.5ml) and THF (50ml). The mixture was stirred overnight at room temperature after which time most of the solvent was removed in vacuo and the residue partitioned between water (containing 2N sodium hydroxide solution to about pH 12) and ether. The aqueous layer was neutralised with saturated ammonium chloride solution and extracted with ethyl acetate. The organic extracts were combined, backwashed with water and brine, dried and concentrated in vacuo to give the title compound as a white solid (2.95g).
T.l.c. dichloromethane/methanol (10:1), Rf 0.58.

Intermediate 9b

1-[[3-Bromo-2-[2-[2-(triphenylmethyl)-2H-tetrazol-5-yl]phenyl]-5-benzofuranyl]methyl]-4-chloro-2-propyl-1H-imidazole-5-carboxylic acid

Intermediate 9b was prepared from 4-chloro-2-propyl-1H-imidazole-5-carboxaldehyde in a similar manner to Intermediate 9a above.

Intermediate 9c

1-[[3-Bromo-2-[2-[2-(triphenylmethyl)-2H-tetrazol-5-yl]phenyl]-5-benzofuranyl]methyl]-4-chloro-2-ethyl-1H-imidazole-5-carboxylic acid

Intermediate 9c was prepared from 4-chloro-2-ethyl-1H-imidazole-5-carboxaldehyde in a similar manner to Intermediate 9a above.

Intermediate 10

N-[[1-[[3-Bromo-2-[2-[2-(triphenylmethyl)-2H-tetrazol-5-yl]phenyl] -5-benzofuranyl]methyl]-2-butyl-4-chloro-1H-imidazol-5-yl]carbonyl] glycine ethyl ester

CDI (306mg) was added to a stirred solution of Intermediate 9a (500mg) in dry THF (5ml) at 20°C. The resultant solution was stirred for 21.5h, then glycine ethyl ester hydrochloride (523mg) and triethylamine (0.56ml) were added and the suspension was stirred for 7h. The reaction mixture was concentrated in vacuo to yield an opaque wax (2.61g) which was purified by column chromatography eluting with System B (5:1) to give the title compound as a white solid (575mg).
T.l.c System B(5:1)Rf 0.60

Intermediates 11 to 23 were prepared in a similar manner from Intermediates 9a, 9b or 9c and the corresponding amino acid ester hydrochloride:-

Intermediate 11

N-[[1-[[3-Bromo-2-[2-[2-(triphenylmethyl)-2H-tetrazol-5-yl]phenyl]-5-benzofuranyl]-4-chloro-2-propyl-1H-imidazol-5-yl]carbonyl]glycine ethyl ester

T.l.c. ether Rf 0.63

Intermediate 12

N-[[1-[[3-Bromo-2-[2-[2-(triphenylmethyl)-2H-tetrazol-5-yl]phenyl]-5-benzofuranyl]-4-chloro-2-propyl-1H-imidazol-5-yl]carbonyl]-$\beta$-alanine ethyl ester

T.l.c. System A (4:1) Rf 0.50

Intermediate 13

N-[[1-[[3-Bromo-2-[2-[2-(triphenylmethyl)-2H-tetrazol-5-yl]phenyl]-5-benzofuranyl]-4-chloro-2-ethyl-1H-imidazol-5-yl]carbonyl]glycine methyl ester

T.l.c. ether Rf 0.3

Intermediate 14

N-[[1-[[3-Bromo-2-[2-[2-(triphenylmethyl)-2H-tetrazol-5-yl]phenyl]-5-benzofuranyl]-2-butyl-4-chloro-1H-imidazol-5-yl]carbonyl]-L-alanine ethyl ester

T.l.c. System D (20:1) Rf 0.38

Intermediate 15

N-[[1-[[3-Bromo-2-[2-[2-(triphenylmethyl)-2H-tetrazol-5-yl]phenyl]-5-benzofuranyl]-2-butyl-4-chloro-1H-imidazol-5-yl]carbonyl]-D-alanine ethyl ester

T.l.c. System D (20:1) Rf 0.38

Intermediate 16

N-[[1-[[3-Bromo-2-[2-[2-(triphenylmethyl)-2H-tetrazol-5-yl]phenyl-5-benzofuranyl]-2-butyl-4-chloro-1H-imidazol-5-yl]carbonyl]-L-serine ethyl ester

T.l.c. ether Rf 0.36.

Intermediate 17

N-[[1-[[3-Bromo-2-[2-[2-(triphenylmethyl)-2H-tetrazol-5-yl]phenyl]-5-benzofuranyl]-2-butyl-4-chloro-1H-imidazol-5-yl]carbonyl]-D-serine ethyl ester

T.l.c. ether Rf 0.36

Intermediate 18

N-[[1-[[3-Bromo-2-[2-[2-(triphenylmethyl)-2H-tetrazol-5-yl]phenyl]-5-benzofuranyl]-2-butyl-4-chloro-1H-imidazol-5-yl]carbonyl]glycine methyl ester

T.l.c. System D (30:1) Rf 0.30.

Intermediate 19

N-[[1-[[3-Bromo-2-[2-[2-(triphenylmethyl)-2H-tetrazol-5-yl-1-phenyl]-5-benzofuranyl]-2-butyl-4-chloro-1H-imidazol-5-yl]carbonyl]glycinamide

T.l.c. ether:ethanol (10:1) Rf 0.26.

Intermediate 20

N-[[1-[[3-Bromo-2-[2-[2-(triphenylmethyl)-2H-tetrazol-5-yl]phenyl]-5-benzofuranyl]-4-chloro-2-ethyl-1H-imidazol-5-yl]carbonyl]glycine ethyl ester

T.l.c. System A (3:1) Rf 0.58.

Intermediate 21

N-[[1-[[3-Bromo-2-[2-[2-(triphenylmethyl)-2H-tetrazol-5-yl]phenyl]-5-benzofuranyl]methyl]-2-butyl-4-chloro-1H-imidazol-5-yl]acetyl]glycine ethyl ester

T.l.c. System A (5:1) Rf 0.48

Intermediate 22

N-Methyl-[[1-[[3-bromo-2-[2-[2-(triphenylmethyl)-2H-tetrazol-5-yl]phenyl]-5-benzofuranyl]methyl]-2-butyl-4-chloro-1H-imidazol-5-yl]carbonyl]glycine ethyl ester

T.l.c. System A Rf 0.30

Intermediate 23

N-[[1-[[3-Bromo-2-[2-[2-(triphenylmethyl)-2H-tetrazol-5-yl]phenyl]-5-benzofuranyl]methyl]-4-chloro-2-ethyl-1H-imidazol-5-yl]carbonyl]-L-alanine ethyl ester

T.l.c. ether Rf 0.43

Intermediate 24

1-[[1-[[3-Bromo-2-[2-(1H-tetrazol-5-yl)phenyl]-5-benzofuranyl] methyl]-2-butyl-4-chloro-1H-imidazol-5-yl]-carbonyl]-1H-imidazole

CDI (0.36g) was added to a stirred solution of the acid (9a, 1.50g), in freshly distilled THF (20ml). After stirring for 4h., the solution was concentrated in vacuo to afford a colourless glass. This glass was then subjected to flash column chromatography on silica gel, using ether as the eluent. This gave the title compound as a white powder (1.20g)
T.l.c. System A (5:1) Rf 0.10

Intermediate 25

N-[[1-[[3-Bromo-2-[2-[2-(triphenylmethyl)-2H-tetrazol-5-yl]phenyl]-5-benzofuranyl]methyl]-2-butyl-4-chloro-1H-imidazol-5-yl]carbonyl]-L-phenyl alanine ethyl ester

(L)-Phenylalanine ethyl ester hydrochloride (0.80g) and triethylamine (0.36g) were added to a stirred solution of Intermediate 24 (0.60g) in THF (20ml). The resulting suspension was heated at reflux under nitrogen for 4 days, then cooled and filtered. The filtrate was concentrated in vacuo to give a colourless oil, which was purified by flash column chromatography on silica gel, using System A (1:1) as eluent, to give the title compound as a white powder (270mg).

| | |
|---|---|
| Assay Found<br>$C_{54}H_{47}BrClN_7O_4$ requires | C,66.7;H,4.8;N,9.8.<br>C,66.6;H,4.9;N,10.1%. |

Intermediate 26

N-[[1-[[3-Bromo-2-[2-[2-(triphenylmethyl)-2H-tetrazol-5-yl]phenyl]-5-benzofuranyl]methyl]-2-butyl-4-chloro-1H-imidazol-5-yl]carbonyl]-L-isoleucine ethyl ester

This compound was prepared from Intermediate 24 and (L)-isoleucine in the manner described for the preparation of Intermediate 25 above.

| | |
|---|---|
| Assay found<br>$C_{51}H_{49}BrClN_7O_4$ requires | C,65.5;H,4.9;N,10.15.<br>C,65.2;H,5.3; N,10.4% |

Intermediate 27

1-[[3-Bromo-2-[2-[[(1,1-dimethylethoxy)carbonyl]amino]phenyl]-5-benzofuranyl]methyl]-4-chloro-2-ethyl-1H-imidazole-5-carboxaldehyde

A mixture of 1,1-dimethyethyll [2-[3-bromo-5-(bromomethyl)-2-benzofuranyl]phenyl]carbamate (9.5g) and 4-chloro-2-ethyl-1H-imidazole-5-carboxaldehyde (2.95g) (which are both described in European patent Specification No. 0 434 249, published 26th June 1991) and potassium carbonate (3.1g) in DMF (30ml) was stirred at room temperature under a nitrogen atmosphere for 18h. The suspension was diluted with water (50ml) and extracted with ether (3x150ml). The combined extracts were dried and concentrated in vacuo to an oil which was purified by flash chromatography eluting with System A (3:7) to give the title compound - (4.55g) as a white solid, m.p. 65-66°.
Similarly prepared were:

Intermediate 28

1-[[3-Bromo-2-[2-1[[(1,1-dimethylethoxy)carbonyl]amino]phenyl]-5-benzofuranyl]methyl]-4-chloro-2-propyl-1H-imidazole-5-carboxaldehyde

m.p. 75-76°C
From 1,1-dimethylethyl [2-[3-bromo-5-(bromomethyl)-2-benzofuranyl]phenyl]carbamate and 4-chloro-2-propyl-1H-imidazole-5-carboxaldehyde.

Intermediate 29

1-[[3-Bromo-2-[2-(methoxycarbonyl)phenyl]-5-benzofuranyl]methyl]-2-butyl-4-chloro-1H-imidazole-5-carboxaldehyde

T.l.c. dichloromethane:ethanol:ammonia (300:8:1) Rf 0.8 From methyl 2-[3-bromo-5-(bromomethyl)-2-benzofuranyl]benzoate and 2-butyl-4-chloro-1H-imidazole-5-carboxaldehyde (both described in EP-A-0 434 249).

Intermediate 30

1-[[3-Bromo-2-[2-(methoxycarbonyl)phenyl]-5-benzofuranyl]methyl]-2-ethyl-4-methyl-1H-imidazole-5-carboxaldehyde

m.p. 58°C (softens).
From methyl 2-[3-bromo-5-(bromomethyl)-2-benzofuranyl]benzoate and 2-ethyl-4-methyl-1H-imidazole-5-carboxaldehyde (both described in EP-A 0 434 249).

Intermediate 31

1-[[3-Bromo-2-[2-[[(1,1-dimethylethoxy)carbonyl]amino]phenyl]-5-benzofuranyl]methyl]-4-chloro-2-ethyl-1H-imidazole-5-carboxylic acid

A solution of Intermediate 27 (4.25g) and 2-methyl-2-butene (2M in THF; 45.6ml) in dry THF (125ml) and t-butanol (29ml), under nitrogen atmosphere, was treated with a solution of sodium chlorite (80%; 6.87g) and sodium dihydrogen phosphate dihydrate (6.87g) in water (100ml). The mixture was stirred at room temperature for 20h. The layers were separated and the aqueous layer was extracted with ether (3x100ml). The combined extracts were washed with water (200ml), dried and concentrated in vacuo to give a yellow foam (4.8g). This material was dissolved in 2M sodium hydroxide solution (50ml) and then extracted once with ether (50ml). The basic layer was acidified to pH3 using 2M HCl. The resultant solid was collected by filtration to give the title compound (3.8g) as a white solid. T.l.c.dichloromethane:ether:acetic acid (10:2.5:0.5) Rf = 0.78
Similarly prepared were:-

Intermediate 32

1-[[3-Bromo-2-[2-[[(1,1-dimethylethoxy)carbonyl]amino]phenyl]-5-benzofuranyl]methyl]-4-chloro-2-propyl-1H-imidazole-5-carboxylic acid

T.l.c. dichloromethane:ether:acetic acid (10:2.5:0.5) Rf = 0.78 From Intermediate 28.

Intermediate 33

1-[[3-Bromo-2-[2-(methoxycarbonyl)phenyl]-5-benzofuranyl]methyl] -2-butyl-4-chloro-1H-imidazole-5-carboxylic acid

T.l.c. ether Rf = 0.1
From Intermediate 29.

Intermediate 34

1-[[3-Bromo-2-[2-(methoxycarbonyl)phenyl]-5-benzofuranyl]methyl]-2-ethyl-4-methyl-1H-imidazole-5-carboxylic acid

m.p. 185°C.
From intermediate 30

Intermediate 35

Ethyl 1-[[3-bromo-2-[2-[[(1,1-dimethylethoxy)carbonyl]amino]phenyl]-5-benzofuranyl]methyl]-4-chloro-2-propyl-1H-imidazole-5-carboxylate

A solution of DEAD (1.09ml) in dry THF (10ml) was added, dropwise, at room temperature under nitrogen to a solution of Intermediate 32 (2.7g) and triphenylphosphine (1.8g) in ethanol (0.81ml) and dry THF (120ml). The resulting solution was stirred at room temperature for 4h and then concentrated in vacuo. The residue was purified by flash chromatography eluting with System A (3:7) to give the title compound (2.45g) as a white solid. m.p. 68-70°C.

Intermediate 36

Ethyl 1-[[2-(2-aminophenyl)-3-bromo-5-benzofuranyl]methyl]-4-chloro-2-propyl-1H-imidazole-5-carboxylate

A solution of Intermediate 35 (2.4g) in dry dichloromethane (60ml), under nitrogen, was cooled to 0°C and trifluoroacetic acid (12ml) was added. The resulting solution was stirred at room temperature for 2h. Water (20ml) was added and then aqueous ammonia (20ml). The layers were separated and the organic phase dried and concentrated in vacuo. The residue was purified by flash chromatography eluting with ether to

give the title compound (1.86g) as a white solid. m.p. 45-46°C.

Intermediate 37

Ethyl 1-[[3-bromo-2-[2-[[(trifluoromethyl)sulphonyl]amino]phenyl]-5-benzofuranyl]methyl]-4-chloro-2-propyl-1H-imidazole-5-carboxylate

The title compound was obtained as a white solid (1.7g) from a solution of Intermediate 36 (1.8g) and dry triethylamine (0.49ml) in dry dichloromethane (70ml), according to the method of Example 28. m.p. 77-78°C.

Intermediate 38

1-[[3-Bromo-2-[2-[[(trifluoromethyl)sulphonyl]amino]phenyl]-5-benzofuranyl]methyl]-4-chloro-2-propyl-1H-imidazole-5-carboxylic acid

The title compound was obtained as a white solid (1.3g) from a solution of Intermediate 37 (1.45g) in methanol (60ml) and 2N NaOH (12ml), according to the method of Example 30. m.p. 174-175°C.

Intermediate 39

Phenylmethyl 2-(3-bromo-5-methyl-2-benzofuranyl)benzoate

A solution of DEAD (7.51g) in THF (50ml) was added, dropwise, to a stirred solution of 2-(3-bromo-5-methyl-2-benzofuranyl)benzoic acid (described in EP-A-0 434 249) (8.35g) in THF (200ml) containing triphenylphosphine (11.9g) and benzyl alcohol (2.86ml). The solution was stirred overnight and partitioned between ethyl acetate (200ml) and water (200ml). The organic phase was washed with water (200ml), dried and concentrated in vacuo to give an orange oil. Purification by column chromatogrpahy eluting with System A (1:4) afforded the title compound (8.9g) as a pale yellow gum.
nmr ($CDCl_3$) δ 2.5 (s, 3H), 5.15 (s,2H), 7.0-7.3(m,8H), 7.5-7.65(m,2H), 7.7-7.75(dd,1H), 7.96-803 (dd,1H).

Intermediate 40

Phenylmethyl 2-[3-bromo-5-(bromomethyl)-2-benzofuranyl]benzoate

The title compound was obtained as a pale yellow gum (1.45g) from a stirred solution of Intermediate 39 (4.3g), NBS (2.03g) and dibenzoyl peroxide (0.1g) in carbon tetrachloride (200ml), according to the method of Intermediate 6.
T.l.c. System A (1:1) Rf = 0.5.

Intermediate 41

1-[[3-Bromo-2-[2-[(phenylmethoxy)carbonyl]phenyl]-5-benzofuranyl]methyl]-2-butyl-4-chloro-1H-imidazole-5-carboxaldehyde

The title compound was obtained as a white foam (1.47g) from a solution of Intermediate 40 (1.90g) in DMF (15ml) which was added dropwise to a stirred suspension of Intermediate 7 (0.67g) and anhydrous potassium carbonate (0.59g) in DMF (40ml), according to the method of Intermediate 8.
T.l.c. dichloromethane:ethanol:ammonia (300:8:1) Rf = 0.8.

Intermediate 42

1-[[3-Bromo-2-[2-[(phenylmethoxy)carbonyl]phenyl]-5-benzofuranyl]methyl]-2-butyl-4-chloro-1H-imidazole-5-carboxylic

A solution of sodium chlorite (1.34g) and sodium dihydrogen orthophosphate (1.49g) in water (20ml) was added, dropwise, to a stirred solution of Intermediate 41 (1.45g) and 2-methyl-2-butene (2M in THF; 10ml) in THF (35ml) and tert-butanol (20ml). After vigorous stirring for 20h, the mixture was added to water (40ml)

and ethyl acetate (30ml). The separated aqueous phase was further extracted with ethyl acetate (40ml) and the combined organic extracts were washed with water (2 x 50 ml), dried and concentrated in vacuo to give a viscous colourless oil (1.65g). Purification by column chromatography eluting with a gradient of ether to System E (99:1) afforded the title compound (1.44g) as a brown foam.
n.m.r (DMSO-$d_6$) δ 0.82 (t,3H), 1.3(m,2H), 1.58 (m,2H), 2.69 (t,2H), 5.15 (s,2H), 5.8 (s,2H), 7.0-7.3 (m,7H), 7.58(d,1H), 7.7-7.85 (m,3H), 8.0(d,1H).

Intermediate 43

1-[[3-Bromo-2-[2-[2-(triphenylmethyl)-2H-tetrazol-5-yl]phenyl]-5-benzofuranyl]methyl]-2-butyl-4-methyl-1H-imidazole-5-carboxylic acid

Triethylamine (0.3ml) and trityl chloride (0.42g) were added to a mixture of 1-[[3-bromo-2-[2-(1H-tetrazol-5-yl)phenyl]-5-benzofuranyl]methyl]-2-butyl-4-methyl-1H-imidazole-5-carboxylic acid (described in EP-A- 0 434 249) (400mg) in dichloromethane (20ml). DMAP (5mg) was added and the solution stirred overnight. Water (15ml) was added and the mixture stirred vigorously for 5 minutes. the separated aqueous phase was extracted with dichloromethane (2 x 20ml) and the extracts dried and evaporated in vacuo. Purification by chromatography eluting with dichloromethane:methanol:acetic acid (90:5:5) gave the title compound - (270mg) as a white foam.
T.l.c. dichloromethane:methanol:acetic acid (90:5:5) Rf = 0.47.

Intermediates 44 to 50 were prepared according to the method of Example 23 described below:

Intermediate 44

N-[[1-[[3-Bromo-2-[2-[[(1,1-dimethylethoxy)carbonyl]amino]phenyl]-5-benzofuranyl]methyl]-4-chloro-2-ethyl-1H-imidazol-5-yl]carbonyl] glycine ethyl ester

T.l.c. ether Rf = 0.4
From Intermediate 31 and glycine ethyl ester hydrochloride.

Intermediate 45

N-[[1-[[3-Bromo-2-[2-[2-(triphenylmethyl)-2H-tetrazol-5-yl]phenyl]-5-benzofuranyl]methyl]-2-butyl-4-methyl-1H-imidazol-5-yl]carbonyl]glycine ethyl ester

T.l.c ethyl acetate Rf = 0.3
From Intermediate 43 and glycine ethyl ester hydrochloride.

Intermediate 46

N-[[1-[[3-Bromo-2-[2-[2-(triphenylmethyl)-2H-tetrazol-5-yl]phenyl]-5-benzofuranyl]methyl]-2-ethyl-4-methyl-1H-imidazol-5-yl]carbonyl]glycine ethyl ester

m.p. 80-83°C.
From 1-[[3-bromo-2-[2-[2-(triphenylmethyl)-2H-tetrazol-5-yl]phenyl]-5-benzofuranyl]methyl]-2-ethyl-4-methyl-1H-imidazole-5-carboxylic acid (described in EP-A 0 434 249) and glycine ethyl ester hydrochloride.

Intermediate 47

N-[[1-[[3-Bromo-2-[2-[2-(triphenylmethyl)-2H-tetrazol-5-yl]phenyl]-5-benzofuranyl]methyl]-4-chloro-2-ethyl-1H-imidazol-5-yl]carbonyl]-L-isoleucine ethyl ester

m.p. 76-78°C
From Intermediate 9c and L-isoleucine ethyl ester hydrochloride.

Intermediate 48

1-[[1-[[3-Bromo-2-[2-[2-(triphenylmethyl)-2H-tetrazol-5-yl]phenyl]-5-benzofuranyl]methyl]-2-butyl-4-chloro-1H-imidazol-5-yl]carbonyl]-DL-proline ethyl ester

T.l.c. ether Rf = 0.60
From Intermediate 9a and DL-proline ethyl ester.

Intermediate 49

N-[[1-[[3-Bromo-2-[2-[2-(triphenylmehtyl)-2H-tetrazol-5-yl]phenyl]-5-benzofuranyl]methyl]-2-ethyl-4-methyl-1H-imidazol-5-yl]carbonyl]glycine butyl ester

m.p. 53-55°C
From 1-[[3-bromo-2-[2-[2-(triphenylmethyl)-2H-tetrazol-5-yl]phenyl]-5-benzofuranyl]methyl]-2-ethyl-4-methyl-1H-imidazole-5-carboxylic acid (described in EP-A-0 434 249) and glycine butyl ester hydrochloride.

Intermediate 50

N-[1-[[3-Bromo-2-[2-[2-(triphenylmethyl)-2H-tetrazol-5-yl]phenyl]-5-benzofuranyl]methyl]-4-chloro-2-ethyl-1H-imidazol-5-yl]carbonyl]-glycine butyl ester

T.l.c. ether Rf = 0.72
From Intermediate 9c and glycine butyl ester hydrochloride.

Intermediate 51

N-[[1-[[(2-aminophenyl)-3-bromo-2-5-benzofuranyl]methyl]-4-chloro-2-ethyl-1H-imidazol-5-yl]carbonyl]glycine ethyl ester

A solution of Intermediate 44 (1.1g) in dry dichloromethane (30ml) was treated with trifluoroacetic acid according to the method of Intermediate 36 to give the title compound (0.87g) as a white foam.
T.l.c. ether, Rf = 0.36

Example 1

N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycine ethyl ester

Concentrated hydrochloric acid (0.5ml) was added to a stirred solution of Intermediate 10 (550mg) in ethanol (5ml) and THF (1ml) at 20°C. The solution was stirred at 20°C for 5h, sodium carbonate solution (2N) was added to pH9 and the aqueous mixture was concentrated to a small volume (approx. 1ml). The aqueous solution was washed with ether (3x20ml) then acidified with 2N HCl to pH4 and extracted with ethyl acetate (3x30ml). The combined extracts were dried and concentrated *in vacuo* to yield a straw coloured foam (340mg). Purification by column chromatography eluting with System C (10:1) to gave the title compound as a white foam (257mg).m.p. 108-110°C.
T.l.c. System C (10:1) Rf 0.61

Examples 2 to 14 were prepared in a similar manner from Intermediates 11 to 23, respectively:-

Example 2

N-[[1-[[3-Bromo-2-[2-(1H-tetrazol-5-yl)phenyl]-5-benzofuranyl]methyl]-4-chloro-2-propyl-1H-imidazol-5-yl]-carbonyl]glycine ethyl ester

T.l.c. ether Rf 0.28.

| | |
|---|---|
| Assay Found | C,51.5;H,4.0;N,15.3. |
| $C_{27}H_{25}BrClN_7O_4$ requires | C,51.7; H,4.0; N, 15.6%. |

Example 3

N-[[1-[[3-Bromo-2-[2-(1H-tetrazol-5-yl)phenyl]-5-benzofuranyl]methyl]-4-chloro-2-propyl-1H-imidazol-5-yl]-carbonyl]-β-alanine ethyl ester

m.p. 98°C
T.l.c. ether Rf 0.34.

Example 4

N-[[1-[[3-Bromo-2-[2-(1H-tetrazol-5-yl)phenyl]-5-benzofuranyl]methyl]-4-chloro-2-ethyl-1H-imidazol-5-yl]-carbonyl]glycine methyl ester

m.p. 210-212°C
T.l.c. dichloromethane:ether:ethanol:acetic acid (100:5:5:1) Rf 0.27

Example 5

N-[[1-[[3-Bromo-2-[2-(1H-tetrazol-5-yl)phenyl]-5-benzofuranyl]methyl]-2-butyl-4-chloro-1H-imidazol-5-yl]-carbonyl]-L-alanine ethyl ester

T.l.c. System E (10:1) Rf 0.68.
m.p. 115-120°C

Example 6

N-[[1-[[3-Bromo-2-[2-(1H-tetrazol-5-yl)phenyl]-5-benzofuranyl]methyl]-2-butyl-4-chloro-1H-imidazol-5-yl]-carbonyl]-D-alanine ethyl ester

T.l.c. System D (20:1) Rf 0.38
m.p. 103-107°C

Example 7

N-[[1-[[3-Bromo-2-[2-(1H-tetrazol-5-yl)phenyl]-5-benzofuranyl]methyl]-2-butyl-4-chloro-1H-imidazol-5-yl]-carbonyl]-L-serine ethyl ester

T.l.c. System E (20:1) Rf 0.44
m.p.105-110°C

Example 8

N-[[1-[[3-Bromo-2-[2-(1H-tetrazol-5-yl)phenyl]-5-benzofuranyl]methyl]-2-butyl-4-chloro-1H-imidazol-5-yl]-carbonyl]-D-serine ethyl ester

T.l.c. System E (20:1) Rf 0.44
m.p. 77-83°C.

Example 9

N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycine methyl ester

mp 112-115°C
T.l.c. dichloromethane:methanol (10:1) Rf 0.53

Example 10

N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycinamide

mp 110°-112°C
T.l.c. ether:ethanol(10:1) Rf 0.26

Example 11

N-[[1-[[3-Bromo-2-[2-(1H-tetrazol-5-yl)phenyl]-5-benzofuranyl]methyl]-4-chloro-2-ethyl-1H-imidazol-5-yl]-carbonyl]glycine ethyl ester

T.l.c. ether Rf. 0.10
IR (Nujol mull) 3305, 1744 and 1653cm$^{-1}$.

Example 12

N-[[1-[[3-Bromo-2-[2-(1H-tetrazol-5-yl)phenyl]-5-benzofuranyl]methyl]-2-butyl-4-chloro-1H-imidazol-5-yl] acetyl]glycine ethyl ester

m.p. 126-130°C.
T.l.c. dichloromethane:methanol(10:1)Rf 0.56

Example 13

N-Methyl-[[1-[[3-bromo-2-[2-(1H - tetrazol-5-yl)phenyl]-5-benzofuranyl)methyl]-2-butyl-4-chloro-1H-imidazol-5-yl]carbonyl]glycine ethyl ester

T.l.c. ether acetate Rf 0.32
IR (Nujol mull) 3400, 1745 and 1650cm$^{-1}$.

Example 14

N-[[1-[[3-Bromo-2-[2-(1H - tetrazol-5-yl)phenyl]-5-benzofuranyl]methyl]-4-chloro-2-ethyl-1H-imidazol-5-yl]-carbonyl]-L-alanine ethyl ester

m.p 108-110°C
T.l.c. ether Rf 0.3
Examples 15 and 16 were prepared in a similar manner from Intermediates 25 and 26 respectively.

Example 15

N-[[1-[[3-Bromo-2-[2-(1H - tetrazol-5-yl)phenyl]-5-benzofuranyl]methyl]-2-butyl-4-chloro-1H - imidazol-5-yl]-carbonyl]-L-phenylalanine ethyl ester

mp 146-148°C
$[\alpha]D^{20}$ (chloroform, c = 7mg/ml) = +15.96°

Example 16

N-[[1-[[3-Bromo-2-[2-(1H - tetrazol-5-yl)phenyl]-5-benzofuranyl]methyl]-2-butyl-4-chloro-1H-imidazol-5-yl]-carbonyl]-L-isoleucine ethyl ester

mp 133-135°C
$[\alpha]D^{20}$ (chloroform, c = 7mg/ml) = +3.33°

Example 17

N-[[1-[[3-Bromo-2-[2-(1H-tetrazol-5-yl)phenyl]-5-benzofuranyl]methyl]-2-butyl-4-chloro-1H imidazol-5-yl]-

carbonyl]glycine

Potassium hydroxide (39mg) was added to a stirred solution of the product of Example 1 (190mg) in ethanol (1.1ml) and water (0.3ml) at 20 ° C. The resultant mixture was stirred at 20 ° C for 4h. Dilute hydrochloric acid (2N) was added to pH2, the mixture was then extracted with ethyl acetate (3x20ml), dried and concentrated in vacuo to give the title compound as a buff coloured solid (118mg).
T.l.c. dichloromethane:ether:acetic acid (75:25:1) Rf 0.22 IR (Nujol mull) 3600-2400,1733,1654 and 1538cm$^{-1}$.

Examples 18 to 22 inclusive were prepared in a similar manner:-

Example 18

N-[[1-[[3-Bromo-2-[2-(1H - tetrazol-5-yl)phenyl]-5-benzofuranyl]methyl]-4-chloro-2-propyl-1H-imidazol-5-yl]-carbonyl]glycine

From the product of Example 2.
mp 217 ° C
T.l.c. ether:ethanol:acetic acid (90:5:1) Rf 0.35

Example 19

N-[[1-[[3-Bromo-2-[2-(1H - tetrazol-5-yl)phenyl]-5-benzofuranyl]methyl]-4-chloro-2-propyl-1H - imidazol-5-yl]-carbonyl]-β-alanine

From the product of Example 3.
mp 98 ° C
T.l.c. ether:ethanol:acetic acid (90:5:1) Rf 0.46.

Example 20

N-[[1-[[3-Bromo-2-[2-(1H - tetrazol-5-yl)phenyl]-5-benzofuranyl]methyl]-2-butyl-4-chloro-1H-imidazol-5-yl]-carbonyl]-L-alanine
From the product of Example 5.
T.l.c. System E (20:1) Rf 0.54
m.p. 165°C.

Example 21

N-[[1-[[3-Bromo-2-[2-(1H - tetrazol-5-yl)phenyl]-5-benzofuranyl]methyl]-2-butyl-4-chloro-1H-imidazol-5-yl]-carbonyl]-D-alanine

From the product of Example 6.
T.l.c. System E (20:1) Rf 0.54
m.p. 168-170°C.

Example 22

N-[[1-[[3-Bromo-2-[2-(1H - tetrazol-5-yl)phenyl]-5-benzofuranyl]methyl]-4-chloro-2-ethyl-1H-imidazol-5-yl]-carbonyl]glycine

From the product of Example 11.
mp 153-157 ° C
T.l.c. ether:methanol (99:1) Rf 0.15

Example 23

N-[[1-[[3-Bromo-2-[2-[[(trifluoromethyl)sulphonyl]amino]phenyl]-5-benzofuranyl]methyl]-4-chloro-2-propyl-1H-imidazol-5-yl]carbonyl] glycine ethyl ester

CDI (0.527g) was added to a solution of Intermediate 38 (0.7g) in dry THF (35ml) under nitrogen and the solution was stirred at room temperature for 4h. Glycine ethyl ester hydrochloride (0.767g) and triethylamine (0.77ml) were added and the resulting mixture was stirred at room temperature for 20h. After filtration the solution was concentrated in vacuo. The residue was dissolved in ethyl acetate (30ml) and washed with water (2x30ml). The organic layer was dried and concentrated in vacuo. The residue was purified by flash chromatography eluting with ether to give a solid which was further purified by dissolving it in ether (20ml) and washing with water (4x20ml). The organic layer was dried and concentrated in vacuo to give the title compound (0.55g) as a white solid, m.p. 98-100° (dec.).
T.l.c. ether, Rf = 0.63
Similarly prepared were:-

Example 24

Methyl 2-[3-bromo-5-[[2-butyl-4-chloro-5-[[(2-ethoxy-2-oxoethyl)amino]carbonyl]-1H-imidazol-1-yl]methyl]-2-benzofuranyl]benzoate

T.l.c. System A (2:1) Rf = 0.75

| | | | |
|---|---|---|---|
| Assay Found: | C,51.8; | H,4.4; | N,6.0; |
| $C_{29}H_{29}BrClN_3O_6.0.4CHCl_3$ requires | C,52.0; | H,4.4; | N,6.2% |

From Intermediate 33 and glycine ethyl ester hydrochloride.

Example 25

Methyl 2-[3-bromo-5-[[2-butyl-5-[[(2-ethoxy-2-oxoethyl)amino] carbonyl]-4-methyl-1H-imidazol-1-yl]methyl]-2-benzofuranyl]benzoate

m.p. 55°C (softens)
$MH^+$ calc. 582
obs. 582
From Intermediate 34 and glycine ethyl ester hydrochloride.

Example 26

Phenylmethyl 2-[3-bromo-5-[[2-butyl-4-chloro-5-[[(2-ethoxycarbonyl) amino]carbonyl-1H-imidazol-1yl]-methyl]-2-benzofuranyl]benzoate

T.l.c. System A (1:2) Rf = 0.3
n.m.r. ($CDCl_3$) δ 0.9 (t,3H), 1.28(t,3H), 1.38 (m,2H), 1.7 (m,2H), 2.66 (t,2H), 4.16 (d,2H), 4.24 (q,2H), 5.16 (s,2H), 5.76 (s, 2H), 7.0 (dd,1H), 7.06-7.3 (m, 7H), 7.52-7.72 (m,3H), 8.0 (dd,1H).
From Intermediate 42 and glycine ethyl ester hydrochloride.

Example 27

N-[[1-[[3-Bromo-2-[2-[[(trifluoromethyl)sulphonyl]amino]phenyl]-5-benzofuranyl]methyl]-2-butyl-4-chloro-1H-imidazol-5-yl]carbonyl] glycine ethyl ester
m.p. 76°C (softens)

| | | | |
|---|---|---|---|
| Assay Found: | C, 46.6; | H,3.6; | N,7.5 |
| $C_{28}H_{27}BrClF_3N_4O_6S$ requires: | C, 46.7; | H, 3.8; | N,7.8% |

From 1-[[3-bromo-2-[2-[[(trifluoromethyl)sulphonyl]amino]phenyl]-5-benzofuranyl]methyl]-2-butyl-4-chloro-1H-imidazole-5-carboxylic acid (described in EP-A 0 434 249) and glycine ethyl ester hydrochloride.

Example 28

N-[[1-[[3-Bromo-2-[2-[[(trifluoromethyl)sulphonyl]amino]phenyl]-5-benzofuranyl]methyl]-4-chloro-2-ethyl-1H-imidazol-5-yl]carbonyl]glycine ethyl ester

A solution of Intermediate 51 (0.4g) and dry triethylamine (0.1ml) in dry dichloromethane (14ml) was cooled to -95°C, under nitrogen atmosphere, and a solution of trifluoromethanesulphonic anhydride (0.14ml) in dry dichloromethane (1ml) was added. The mixture was stirred at -95° to -75° for 1h. Water (5ml) was added, the layers were separated and the organic phase was washed with 2N HCl (10ml). The organic solution was dried and concentrated in vacuo to give an oil which was purified by flash chromatography eluting with chloroform:methanol (75:2.5) to give the title compound (0.21g) as a beige solid, m.p. 85-7°C (dec.).
T.l.c. chloroform:methanol (15:0.5) Rf = 0.54

Example 29

2-[3-Bromo-5-[[2-butyl-4-chloro-5-[[(2-hydroxy-2-oxoethyl)amino] carbonyl]-1H-imidazol-1-yl]methyl]-2-benzofuranyl]benzoic acid

2N Sodium hydroxide (2ml) was added to a stirred solution of the product of Example 24 (0.3g) in THF (30ml) and methanol (15ml). After stirring for 5h, the pale yellow solution was acidified with 2N hydrochloric acid, brine (15ml) added and was then extracted with ethyl acetate (2x35ml). The combined organic extracts were washed with water (2x50ml), dried and concentrated in vacuo to afford a white foam (0.223g). The foam (0.215g) was redissolved in THF (10ml) and methanol (5ml) before 2N sodium hydroxide (1.5ml) was added. After standing for 60h, the solution was acidified to pH2 with 2N hydrochloric acid and then concentrated in vacuo to give a residue (5ml) which was partitioned between ethyl acetate (20ml) and brine (15ml). The separated organic phase was dried and concentrated in vacuo to afford a white foam which was dried in vacuo to afford the title compound (0.125g) as a white foam, m.p. 105°-109°.

| | | | |
|---|---|---|---|
| Assay Found: | C,52.75; | H,4.2; | N,6.5; |
| $C_{26}H_{23}BrClN_3O_6.0.2C_4H_8O_2$ requires | C,53.1; | H,4.05; | N,6.9% |

Example 30

N-[[1-[[3-Bromo-2-[2-[[(trifluoromethyl)sulphonyl]amino]phenyl]-5-benzofuranyl]methyl]-4-chloro-2-propyl-1H-imidazol-5-yl]carbonyl] glycine

A solution of the product of Example 23 (0.352g) in methanol (15ml) and 2N sodium hydroxide (3ml) was heated at reflux for 1h. The solution was concentrated in vacuo. It was then diluted with water (10ml) and extracted with ether (15ml). The aqueous layer was acidified to pH3 using 2N HCl and extracted with ethyl acetate (3x20ml). The combined organic extracts were dried and concentrated in vacuo to give the title compound (0.29g) as a white solid, m.p. 159-160° (dec.).
T.l.c. dichloromethane:ether:acetic acid (10:2.5:0.5) Rf = 0.43
Similarly prepared was:-

Example 31

N-[[1-[[3-Bromo-2-[2-[[(trifluoromethyl)sulphonyl]amino]phenyl]-5-benzofuranyl]methyl]-4-chloro-2-ethyl-1H-imidazol-5-yl]carbonyl]glycine

m.p. 199-200°C.
T.l.c. dichloromethane:ether:acetic acid (10:2.5:0.5) Rf = 0.42 From a solution of the product of Example 28 (0.26g) in methanol (10ml) and 2N sodium hydroxide (2ml).

Example 32

N-[[1-[[3-Bromo-2-[2-(1H-tetrazol-5-yl)phenyl]-5-benzofuranyl] methyl]-2-butyl-4-methyl-1H-imidazol-5-yl]-carbonyl]glycine ethyl ester

A solution of Intermediate 45 (120mg) in ethanol (5ml) and conc. hydrochloric acid (2ml) was stirred at room temperature for 4h. The solution was basified with 8% sodium bicarbonate solution to pH10 and extracted with ethyl acetate (3x20ml). The dried organic extracts were evaporated in vacuo, and the residue purified by flash chromatography eluting with ether:ethanol:acetic acid (10:2:1) to give the title compound - (65mg) as a white solid. m.p. 180-190°C (dec.)
T.l.c. ether:ethanol:acetic acid (10:2:1) Rf = 0.47
Similarly prepared were:-

Example 33

N-[[1-[[3-Bromo-2-[2-[(1H-tetrazol-5-yl)phenyl]-5-benzofuranyl] methyl]-2-ethyl-4-methyl-1H-imidazol-5-yl]-carbonyl]glycine ethyl ester

m.p. 123-125°C
T.l.c. (5% ethanol in ethyl acetate) Rf = 0.06
From a solution of Intermediate 46 (1.46g) in ethanol (50ml) and conc. hydrochloric acid (0.5ml).

Example 34

N-[[1-[[3-Bromo-2-[2-(1H-tetrazol-5-yl)phenyl]-5-benzofuranyl] methyl]-4-chloro-2-ethyl-1H-imidazol-5-yl]-carbonyl]glycine ethyl ester

m.p. 136-141°C
T.l.c. ether Rf = 0.23
From a solution of Intermediate 47 (120mg) in ethanol (10ml) and conc.hydrochloric acid (0.1ml).

Example 35

1-[[1-[[3-Bromo-2-[2-(1H-tetrazol-5-yl)phenyl]-5-benzofuranyl]methyl]-2-butyl-4-chloro-1H-imidazol-5-yl]-carbonyl]-DL-proline ethyl ester

T.l.c. ether Rf = 0.3

| | | | |
|---|---|---|---|
| Assay found: | C,54.2; | H, 4.6; | N, 14.0 |
| $C_{31}H_{31}BrClN_7O_4$ requires | C, 54.7; | H, 4.6; | N, 14.4% |

From a solution of Intermediate 48 (500mg) in ethanol (15ml) and conc. hydrochloric acid (0.3ml).

Example 36

N-[[1-[[3-Bromo-2-[2-(1H-tetrazol-5-yl)phenyl]-5-benzofuranyl] methyl]-2-ethyl-4-methyl-1H-imidazol-5-yl]-carbonyl]glycine butyl ester

A solution of Intermediate 49 (100mg) and concentrated hydrochloric acid (0.1ml) in t-butanol (10ml) was stirred at room temperature for 16h. The mixture was then basified to pH9 with 8% w/v aqueous sodium hydrogen carbonate (2N) and the solvents then removed in vacuo. The residue was partitioned between water (100ml) and ether (3x50ml), the aqueous phase was acidified to pH5 (2N HCl) and extracted into ethyl acetate (3x40ml). The combined ethyl acetate fractions were dried and concentrated in vacuo to afford the title compound (75mg) m.p. 113-115 ° C.
T.l.c.ethyl acetate:ethanol (95:5) Rf = 0.1
Similarly prepared was:-

Example 37

N-[[1-[[3-Bromo-2-[2-(1H-tetrazol-5-yl)phenyl]-5-benzofuranyl] methyl]-4-chloro-2-ethyl-1H-imidazol-5-yl]-carbonyl]glycine ethyl ester

T.l.c. ether Rf = 0.12
m.p. 75-77°C
From a solution of Intermediate 50 (937mg) in t-butanol (20ml) and conc. hydrochloric acid (0.5ml).

Example 38

N-[[1-[[3-Bromo-2-[2-(1H-tetrazol-5-yl)phenyl]-5-benzofuranyl] methyl]-4-chloro-2-ethyl-1H-imidazol-5-yl]-carbonyl]-L-alanine

A solution of the product of Example 14 (200mg) and lithium hydroxide monohydrate (66.2mg) in water (6ml) and THF (15ml) was stirred at room temperature for 40h before the solution was concentrated in vacuo. The residue was dissolved in water (20ml), acidified to pH5 with 2N hydrochloric acid and the mixture extracted with ethyl acetate (3 x 10ml). The combined extracts were dried and concentrated in vacuo to give the title compound (34mg) as a white powder.
m.p.143-146°C
T.l.c. ethyl acetate Rf = 0.1
Similarly prepared were:-

Example 39

N-[[1-[[3-Bromo-2-[2-(1H-tetrazol-5-yl)phenyl]-5-benzofuranyl] methyl]-2-butyl-4-chloro-1H-imidazol-5-yl]-carbonyl]-L-isoleucine

T.l.c. (5% acetic acid in ether) Rf = 0.54
n.m.r. (250MHz, $CDCl_3$) δ 0.8-1.0 (m,6H), 1.1-1.6 (m, 7H), 1.73 (quint, 2H), 2.0 (m, 1H), 2,74 (t,2H), 4,45 (m,1H), 5,35 & 5.73 (ABq, 2H), 6.4 (d, 1H), 6.9-8.2 (m, 6H).
From a solution of the product of Example 16 (91mg) and lithium hydroxide monohydrate (27mg) in water (2ml) and THF (5ml).

Example 40

N-[[1-[[3-Bromo-2-[2-(1H-tetrazol-5-yl)phenyl]-5-benzofuranyl] methyl]-2-butyl-4-chloro-1H-imidazol-5-yl]-carbonyl]-L-phenylalanine

T.l.c. (5% acetic acid in ether ) Rf = 0.74
n.m.r (250MHz, $CDCl_3$) δ 0.9 (t,3H), 1.4 (hex, 2H), 1.72 (quint, 2H), 2.74 (t,2H), 3.0-3.3 (m,12H), 4.6 (m,1H), 5.27-5.95 (ABq, 2H), 6.8-8.1 (m, 12H).
From a solution of the product of Example 15 (80mg) and lithium hydroxide monohydrate (25mg) in water (2ml) and THF (5ml).

Example 41

2-[3-Bromo-5-[[2-butyl-4-chloro-5-[[(2-ethoxy-2-oxoethyl)amino] carbonyl]-1H-imidazol-1-yl]methyl]-2-benzofuranyl]benzoic acid A mixture of the product of Example 26 (0.71g) and in hydrochloric acid (1ml) in absolute ethanol (40ml) was hydrogenated over 5% palladium on carbon (50% paste, 0.125g) at room temperature and pressure for 2h. After the catalyst had been filtered off, the filtrate was concentrated in vacuo to afford a yellow gum (0.62g). Purification by chromatography eluting with ether:hexane:acetic acid (20:9:1) afforded the title compound (0.26g) as a white foam, m.p. 80°-85° (shrinks 74°-77°).

| | | | |
|---|---|---|---|
| Assay Found: | C,54.05; | H,4.4; | N,6.4; |
| $C_{28}H_{27}BrClN_3O_6$ requires | C,54.5; | H,4.4; | N,6.8% |

Example 42

1-[[1-[[3-Bromo-2-[2-(1H-tetrazol-5-yl)phenyl]-5-benzofuranyl]methyl]-2-butyl-4-chloro-1H-imidazol-5-yl]-

carbonyl]-DL-proline

A solution of the product of Example 35 (130mg) and potassium hydroxide (22mg) in ethanol (2ml) was stirred at room temperature for 4h. The solvent was removed in vacuo and the residue dissolved in water (20ml). After acidification to pH5 (2N HCl) the aqueous layer was extracted with ethyl acetate (3x15ml). The organic extracts were combined, dried and concentrated to afford the title compound (100mg). m.p. 155-160°C.
T.l.c. (dichloromethane:ether:acetic acid, 90:8:2), Rf = 0.08

Example 43

N-[[1-[[3-Bromo-2-[2-(1H-tetrazol-5-yl)phenyl]-5-benzofuranyl] methyl]-2-ethyl-4-methyl-1H-imidazol-5-yl]-carbonyl]glycine

A solution of the product of Example 33 (100mg) and potassium hydroxide (21.9mg) in ethanol (0.6ml) and water (0.15ml) was stirred at room temperature for 16h. The mixture was then acidified to pH6 with 0.1N hydrochloric acid and extracted with chloroform (3 x 5ml). The aqueous phase was acidified to pH2 with 2N hydrochloric acid and extracted with dichloromethane (3 x 5ml). The combined extracts were discarded and the aqueous phase concentrated to dryness and the residue triturated with iso-propanol (3 x 10ml). The combined triturants were concentrated in vacuo to give the title compound (67mg) as a white powder.
m.p. 175-180°C.
T.l.c. (20% ethanol in ethyl acetate) Rf = 0.09.

The compounds of the invention are tested in vitro for angiotensin II antagonism. Aortic strips are obtained from male New Zealand white rabbits and prepared for recording isometric contractions in response to cumulative addition of angiotensin II. The potencies of test antagonists are assessed by measuring their abilities to displace the angiotensin II cumulative concentration response curve. The method used is that of Ackerly et al., Proc. Natl. Acad. Sci., 74(12), pp5725-28 (1977) with the exception that the final composition of the physiological salt solution is as given below in Table 1:

TABLE 1

| Ingredient | Amount (mM) |
|---|---|
| $Na^+$ | 143.4 |
| $K^+$ | 5.9 |
| $Mg^{2+}$ | 0.6 |
| $Ca^{2+}$ | 1.3 |
| $Cl^-$ | 124.5 |
| $HPO^{4-}$ | 1.2 |
| $SO_4^{2-}$ | 0.6 |
| $HCO_3^-$ | 25.0 |
| glucose | 11.1 |
| indomethacin | 0.005 |
| ascorbic acid | 0.1 |

The tissues are initially challenged with $K^+$ (80mM) and then washed at 0, 5, 10 and 15 minutes after the response to $K^+$ has plateaued. After a further 45 minutes an angiotensin II cumulative response curve is constructed (0.1nM to 0.1µM in 10-fold increments) and the tissues are washed as before. A second, third and fourth angiotensin II cumulative response curve (0.1nM to 0.1µM in 3-fold increments) is then constructed at hourly intervals (15 minutes washing after each curve followed by 45 minutes equilibration). The compounds of the invention (30µM) are tested for angiotensin II antagonism by application between 45 and 180 minutes before construction of the fourth angiotensin II curve. The third and fourth angiotensin II curves are expressed graphically and a concentration ratio (CR) is calculated by dividing the angiotensin II $EC_{50}$ value obtained in the presence of the test antagonist (i.e. fourth curve) by the angiotensin II $EC_{50}$ value obtained in the absence of the test antagonist (i.e. third curve).

The potency of the test antagonist is expressed as a pKb which is calculated from the equation :

$$pKb = -\log \left[ \frac{CR-1}{[\text{antagonist}]} \right]$$

which is a rearrangement of equation 4 described by Furchgott, in Handbook of Exp. Pharmacol., 33, p290 (1972) (eds. Blaschko and Muscholl).

If a compound supresses the maximum response to angiotensin II, a pKb is estimated using the double reciprocal plot technique for insurmountable antagonists, described by T.P. Kenakin, Pharmacol. Rev., 36-(3), pp165-222 (esp. 203-204) (1984).

Compounds of the invention will desirably exhibit a pKb in the range between 5 and 12. Thus we have found that the compounds of the invention inhibit the action of the hormone angiotensin II and are therefore useful in the treatment of conditions in which it is desirable to inhibit angiotensin II activity. In particular, the compounds of the Examples are active in the above test.

There is thus provided as a further aspect of the invention a compound of general formula (I) or a physiologically acceptable salt, solvate or metabolically labile ester thereof for use in the treatment of conditions associated with excessive or unregulated angiotensin II activity.

In a further or alternative aspect of the invention there is provided a compound of general formula (I) or a physiologically acceptable salt, solvate or metabolically labile ester thereof for the manufacture of a therapeutic agent for the treatment of conditions associated with excessive or unregulated angiotensin II activity.

There is also provided in a further or alternative aspect of the invention a method for the treatment of conditions associated with excessive or unregulated angiotensin II activity in a mammal including man comprising administration of an effective amount to a mammal in need of such treatment a compound of general formula (I) or a physiologically acceptable salt, solvate or metabolically labile ester thereof.

In addition, by virtue of their antagonistic activity at angiotensin II receptors, compounds of the present invention will be of value in the treatment of conditions associated with activation of the Renin-Angiotensin System.

There is thus provided a further aspect of the present invention a compound of general formula (I) or a physiologically acceptable salt, solvate or metabolically labile ester thereof for use in the treatment of a condition associated with activation of the Renin-Angiotensin system.

In a further or alternative aspect of the present invention there is provided a compound of general formula (I) or a physiologically acceptable salt, solvate or metabolically labile ester thereof for the manufacture of a therapeutic agent for the treatment of a condition associated with activation of the Renin-Angiotensin System.

There is also provided in a further or alternative aspect of the present inventions a method for the treatment of a condition associated with the activation of the Renin-Angiotensin System in a mammal including man comprising administration of an effective amount to a mammal in need of such treatment of a compound of general formula (I) or a physiologically acceptable salt, solvate or metabolically labile ester thereof.

The following examples illustrate pharmaceutical formulations according to the invention. The term "active ingredient" is used herein to represent a compound of formula (I).

Pharmaceutical Example I

| Oral Tablet A | |
|---|---|
| Active Ingredient | 700mg |
| Sodium starch glycollate | 10mg |
| Microcrystalline cellulose | 50mg |
| Magnesium stearate | 4mg |

Sieve the active ingredient and microcrystalline cellulose through a 40 mesh screen and blend in a appropriate blender. Sieve the sodium starch glycollate and magnesium stearate through a 60 mesh screen,

add to the powder blend and blend until homogeneous. Compress with appropriate punches in an automatic tablet press. The tablets may be coated with a thin polymer coat applied by the film coating techniques well known to those skilled in the art. Pigments may be incorporated in the film coat.

Pharmaceutical Example 2

| Oral Tablet B | |
|---|---|
| Active Ingredient | 500mg |
| Lactose | 100mg |
| Maize Starch | 50mg |
| Polyvinyl pyrrolidone | 3mg |
| Sodium starch glycollate | 10mg |
| Magnesium stearate | 4mg |
| Tablet Weight | 667mg |

Sieve the active ingredient, lactose and maize starch through a 40 mesh screen and blend the powders in a suitable blender. Make an aqueous solution of the polyvinyl pyrrolidone (5 - 10% w/v). Add this solution to the blended powders and mix until granulated; pass the granulate through a 12 mesh screen and dry the granules in a suitable oven or fluid bed dryer. Sieve the remaining components through a 60 mesh screen and blend them with the dried granules. Compress, using appropriate punches, on an automatic tablet press.

The tablets may be coated with a thin polymer coat applied by film coating techniques well known to those skilled in art. Pigments may be incorporated in the film coat.

Pharmaceutical Example 3

| Inhalation Cartridge | |
|---|---|
| Active Ingredient | 1mg |
| Lactose | 24mg |

Blend active ingredient, particle size reduced to a very fine particle size (weight mean diameter ca. 5µm) with the lactose in a suitable powder blender and fill the powder blender into No. 3 hard gelatin capsules.

The contents of the cartridges may be administered using a powder inhaler.

Pharmaceutical Example 4

| Injection Formulation | |
|---|---|
| | % w/v |
| Active ingredient | 1.00 |
| Water for injections B.P. to | 100.00 |

Sodium chloride may be added to adjust the tonicity of the solution and the pH may be adjusted to that of maximum stability and/or to facilitate solution of the active ingredient using dilute acid or alkali or by the addition of suitable buffer salts. Antioxidants and metal chelating salts may also be included.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

## Claims

**1.** A compound of the general formula (I):

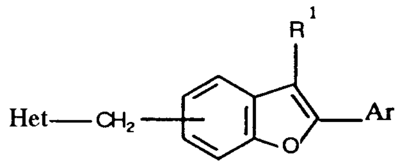

(I)

or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy ,-CHO, $-CO_2H$ or $-COR^2$;
Ar represents the group

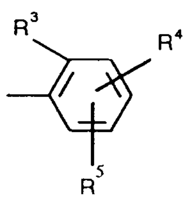

;

$R^2$ represents a group selected from $C_{1-6}$alkyl, $C_{2-6}$alkenyl, $C_{1-6}$alkoxy or the group $-NR^{12}R^{13}$;
$R^3$ represents a group selected from $-CO_2H$, $-NHSO_2CF_3$ or a C-linked tetrazolyl group;
$R^4$ and $R^5$ which may be the same or different each independently represent a hydrogen atom or a halogen atom or a $C_{1-6}$alkyl group;
Het represents the group

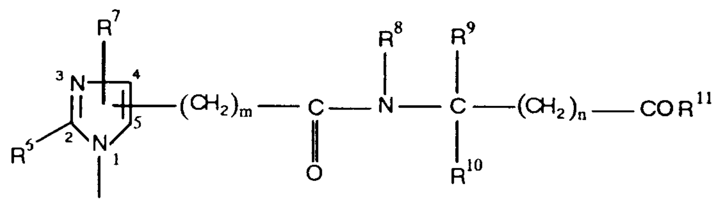

;

$R^6$ represents a hydrogen atom or a group selected from $C_{1-6}$alkyl, $C_{2-6}$alkenyl, $C_{1-6}$alkylthio, $C_{1-6}$alkoxy, $C_{3-7}$cycloalkyl or $C_{3-7}$cycloalkyl$C_{1-4}$alkyl;
$R^7$ represents a hydrogen atom or a halogen atom or a group selected from $C_{1-6}$alkyl, $C_{2-6}$alkenyl or fluoro$C_{1-6}$alkyl;
$R^8$ represents a hydrogen atom or a $C_{1-6}$alkyl group;
$R^9$ represents a hydrogen atom or a group selected from phenyl or $C_{1-6}$alkyl, optionally substituted by a phenyl, hydroxyphenyl, hydroxyl, amino, guanidino, imidazolyl, indolyl, mercapto, $C_{1-6}$alkylthio, carboxyl or amido group; or
$R^8$ and $R^9$, when taken together, form an alkylene bridge of 2, 3 or 4 carbon atoms;
$R^{10}$ represents a hydrogen atom or a $C_{1-6}$alkyl group;
$R^{11}$ represents a group selected from hydroxyl, $C_{1-6}$alkoxy, $C_{2-6}$alkenoxy or $-NR^{12}R^{13}$;
$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents zero or an integer from 1 to 4; and
n represents zero or an integer from 1 to 3; with the proviso that when n represents an integer from 1 to 3, $R^9$ and $R^{10}$ both represent hydrogen atoms.

**2.** A compound as claimed in Claim 1 or a physiologically acceptable salt, solvate or metabolically labile

ester thereof wherein
$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy ,-CHO, $-CO_2H$ or $-COR^2$;
Ar represents the group

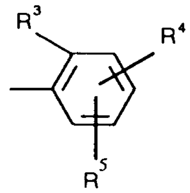

;

$R^2$ represents a group selected from $C_{1-6}$alkyl, $C_{2-6}$alkenyl, $C_{1-6}$alkoxy or the group $-NR^{12}R^{13}$;
$R^3$ represents a group selected from $-CO_2H$, $-NHSO_2CF_3$ or a C-linked tetrazolyl group;
$R^4$ and $R^5$ which may be the same or different each independently represent a hydrogen atom or a halogen atom or a $C_{1-6}$alkyl group;
Het represents the group

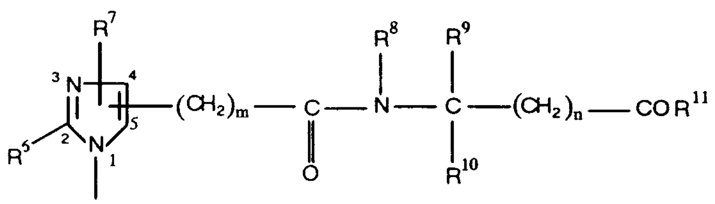

;

$R^6$ represents a hydrogen atom or a group selected from $C_{1-6}$alkyl or $C_{2-6}$alkenyl;
$R^7$ represents a hydrogen atom or a halogen atom or a group selected from $C_{1-6}$alkyl, $C_{2-6}$alkenyl or fluoro$C_{1-6}$alkyl;
$R^8$ represents a hydrogen atom or a $C_{1-6}$alkyl group;
$R^9$ represents a hydrogen atom or a group selected from phenyl or $C_{1-6}$alkyl, optionally substituted by a phenyl, hydroxyphenyl, hydroxyl, amino, guanidino, imidazolyl, indolyl, mercapto, $C_{1-6}$alkylthio, carboxyl or amido group; or
$R^8$ and $R^9$, when taken together, form an alkylene bridge of 2, 3 or 4 carbon atoms;
$R^{10}$ represents a hydrogen atom or a $C_{1-6}$alkyl group;
$R^{11}$ represents a group selected from hydroxyl, $C_{1-6}$alkoxy, $C_{2-6}$alkenoxy or $-NR^{12}R^{13}$;
$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents zero or an integer from 1 to 4; and
n represents zero or an integer from 1 to 3; with the proviso that when n represents an integer from 1 to 3, $R^9$ and $R^{10}$ both represent hydrogen atoms.

3. A compound as claimed in either Claim 1 or Claim 2 wherein $R^9$ represents a hydrogen atom or a $C_{1-4}$alkyl group, for example a methyl group, optionally substituted by hydroxyl, phenyl, $-CO_2H$, amino or $-CONH_2$, or $R^8$ and $R^9$, when taken together, form a propylene ($-(CH_2)_3-$) bridge.

4. A compound as claimed in Claim 1 wherein $R^6$ represents a hydrogen atom or a $C_{1-5}$alkyl or a $C_{3-5}$alkenyl group, preferably a $C_{2-4}$alkyl group, for example an ethyl, n-propyl or n-butyl group, or $R^6$ represents a $C_{3-5}$cycloalkyl or a $C_{3-5}$cycloalkyl$C_{1-2}$alkyl group, for example a cyclopropyl, cyclobutyl or cyclopropylmethyl group.

5. A compound as claimed in Claim 2 wherein $R^6$ represents a hydrogen atom or a $C_{1-5}$alkyl or $C_{3-5}$alkenyl group, preferably a $C_{2-4}$alkyl group, for example an ethyl, n-propyl or n-butyl group.

6. A compound as claimed in any one of Claims 1 to 5 wherein $R^7$ represents a halogen atom, preferably

a chlorine atom, or a $C_{1-4}$alkyl group, for example a methyl group.

**7.** A compound as claimed in any one of Claims 1 to 6 wherein $R^7$ is attached to the imidazole ring at the 4-position.

**8.** A compound as claimed in any one of Claims 1 to 7 wherein $R^8$ represents a hydrogen atom.

**9.** A compound as claimed in any one of Claims 1 to 8 wherein $R^{10}$ represents a hydrogen atom.

**10.** A compound as claimed in any one of Claims 1 to 9 wherein $R^{11}$ represents a hydroxyl group or a $C_{1-4}$alkoxy group, for example a methoxy, ethoxy or propoxy group.

**11.** A compound as claimed in any one of Claims 1 to 10 wherein the group Het-$CH_2$- is attached at the 5-position on the benzofuran ring.

**12.** A compound as claimed in any one of Claims 1 to 11 wherein $R^1$ represents a hydrogen atom or a halogen atom or a group selected from $C_{1-6}$alkyl, $C_{1-6}$alkoxy or fluoro$C_{1-6}$alkyl, preferably a bromine atom.

**13.** A compound as claimed in any one of Claims 1 to 12 wherein $R^3$ represents a C-linked tetrazolyl group.

**14.** A compound as claimed in any one of Claims 1 to 13 wherein $R^4$ and $R^5$ each independently represent a hydrogen atom.

**15.** A compound as claimed in Claim 1 or a physiologically acceptable salt, solvate or metabolically labile ester thereof wherein
$R^1$ represents a halogen atom;
Ar represents the group

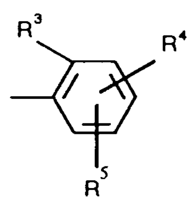

;

$R^3$ represents a group selected from -$CO_2H$, -$NHSO_2CF_3$ or a C-linked tetrazolyl group;
$R^4$ and $R^5$ each independently represent a hydrogen atom;
Het represents the group

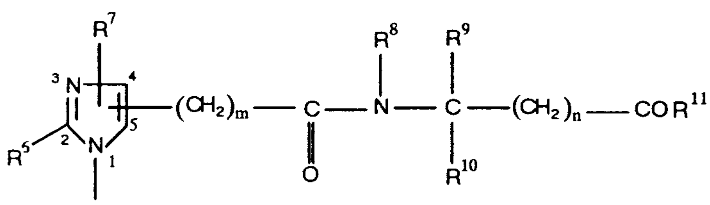

;

$R^6$ represents a $C_{1-6}$alkyl group;
$R^7$ represents a halogen atom or a $C_{1-6}$alkyl group;
$R^8$ represents a hydrogen atom or a $C_{1-6}$alkyl group;
$R^9$ represents a hydrogen atom or a $C_{1-6}$alkyl group, optionally substituted by a phenyl or hydroxyl group, or
$R^8$ and $R^9$, when taken together, form a propylene (-$(CH_2)_3$-) bridge;
$R^{10}$ represents a hydrogen atom;

$R^{11}$ represents a group selected from hydroxyl, $C_{1-6}$alkoxy or $-NH_2$;
m represents zero or an integer from 1 to 4; and
n represents zero or an integer from 1 to 3; with the proviso that when n represents an integer from 1 to 3, $R^9$ and $R^{10}$ both represent hydrogen atoms.

**16.** A compound as claimed in Claim 1 selected from
N-[[1-[[3-Bromo-2-[2-(1H-tetrazol-5-yl)phenyl]-5-benzofuranyl]methyl]-4-chloro-2-ethyl-1H-imidazol-5-yl]-carbonyl]glycine methyl ester;
N-[[1-[[3-Bromo-2-[2-(1H-tetrazol-5-yl)phenyl]-5-benzofuranyl]methyl]-4-chloro-2-ethyl-1H-imidazol-5-yl]-carbonyl]glycine ethyl ester;
N-[[1-[[3-Bromo-2-[2-(1H - tetrazol-5-yl)phenyl]-5-benzofuranyl]methyl]-4-chloro-2-ethyl-1H-imidazol-5-yl]carbonyl]-L-alanine ethyl ester;
N-[[1-[[3-Bromo-2-[2-(1H - tetrazol-5-yl)phenyl]-5-benzofuranyl]methyl]-4-chloro-2-propyl-1H-imidazol-5-yl]carbonyl]glycine;
N-[[1-[[3-Bromo-2-[2-(1H-tetrazol-5-yl)phenyl]-5-benzofuranyl]methyl]-2-butyl-4-chloro-1H-imidazol-5-yl]-carbonyl]-L-alanine ethyl ester;
N-[[1-[[3-Bromo-2-[2-(1H-tetrazol-5-yl)phenyl]-5-benzofuranyl]methyl]-2-butyl-4-chloro-1H-imidazol-5-yl]-carbonyl]-D-alanine ethyl ester;
N-[[1-[[3-Bromo-2-[2-[[(trifluoromethyl)sulphonyl]amino]phenyl]-5-benzofuranyl]methyl]-2-butyl-4-chloro-1H-imidazol-5-yl]carbonyl] glycine ethyl ester;
2-[3-Bromo-5-[[2-butyl-4-chloro-5-[[(2-hydroxy-2-oxoethyl)amino] carbonyl]-1H-imidazol-1-yl]methyl]-2-benzofuranyl]benzoic acid; N-[[1-[[3-Bromo-2-[2-[[(trifluoromethyl)sulphonyl]amino]phenyl]-5-benzofuranyl]methyl]-4-chloro-2-propyl-1H-imidazol-5-yl]carbonyl] glycine;
N-[[1-[[3-Bromo-2-[2-[[(trifluoromethyl)sulphonyl]amino]phenyl]-5-benzofuranyl]methyl]-4-chloro-2-ethyl-1H-imidazol-5-yl]carbonyl]glycine;
N-[[1-[[3-Bromo-2-[2-(1H-tetrazol-5-yl)phenyl]-5-benzofuranyl] methyl]-2-butyl-4-chloro-1H-imidazol-5-yl]carbonyl]-L-isoleucine;
N-[[1-[[3-Bromo-2-[2-(1H-tetrazol-5-yl)phenyl]-5-benzofuranyl] methyl]-2-butyl-4-chloro-1H-imidazol-5-yl]carbonyl]-L-phenylalanine;
N-[[1-[[3-Bromo-2-[2-(1H - tetrazol-5-yl)phenyl]-5-benzofuranyl] methyl]-4-chloro-2-ethyl-1H-imidazol-5-yl]carbonyl]glycine;
or a physiologically acceptable salt, solvate or metabolically labile ester thereof.

**17.** A compound as claimed in Claim 1 selected from
N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycine methyl ester;
N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycine ethyl ester;
N-[[1-[[3-Bromo-2-[2-(1H-tetrazol-5-yl)phenyl]-5-benzofuranyl]methyl]-4-chloro-2-propyl-1H-imidazol-5-yl]carbonyl]glycine ethyl ester;
N-[[1-[[3-Bromo-2-[2-(1H-tetrazol-5-yl)phenyl]-5-benzofuranyl]methyl]-2-butyl-4-chloro-1H imidazol-5-yl]-carbonyl]glycine;
N-[[1-[[3-Bromo-2-[2-(1H-tetrazol-5-yl)phenyl]-5-benzofuranyl]methyl]-4-chloro-2-propyl-1H-imidazol-5-yl]carbonyl]-b-alanine ethyl ester;
N-[[1-[[3-Bromo-2-[2-(1H-tetrazol-5-yl)phenyl]-5-benzofuranyl]methyl]-2-butyl-4-chloro-1H-imidazol-5-yl]-carbonyl]glycinamide;
N-[[1-[[3-Bromo-2-[2-(1H-tetrazol-5-yl)phenyl]-5-benzofuranyl]methyl]-2-butyl-4-chloro-1H imidazol-5-yl]-carbonyl]glycine;
N-[[1-[[3-Bromo-2-[2-[[(trifluoromethyl)sulphonyl]amino]phenyl]-5-benzofuranyl]methyl]-4-chloro-2-propyl-1H-imidazol-5-yl]carbonyl] glycine ethyl ester;
N-[[1-[[3-Bromo-2-[2-(1H-tetrazol-5-yl)phenyl]-5-benzofuranyl] methyl]-4-chloro-2-ethyl-1H-imidazol-5-yl]carbonyl]-L-alanine;
N-[[1-[[3-Bromo-2-[2-(1H-tetrazol-5-yl)phenyl]-5-benzofuranyl] methyl]-2-ethyl-4-methyl-1H-imidazol-5-yl]carbonyl]glycine;
or a physiologically acceptable salt, solvate or metabolically labile ester thereof.

**18.** A process for the preparation of a compound as claimed in any one of Claims 1 to 17 or a physiologically acceptable salt, solvate or metabolically labile ester thereof which comprises:

(A) reacting a benzofuran of general formula (II)

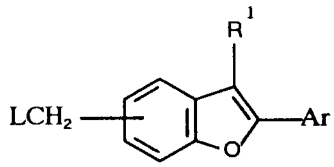

(II)

in which L is a a leaving group, with an imidazole of formula (III)

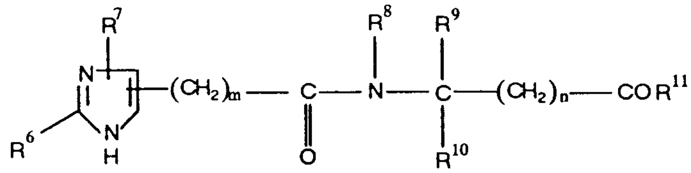

(III)

in which
$R^6$ represents a hydrogen atom or a group selected from $C_{1-6}$alkyl, $C_{2-6}$alkenyl, $C_{1-6}$alkylthio, $C_{1-6}$alkoxy, $C_{3-7}$cycloalkyl or $C_{3-7}$cycloalkyl$C_{1-4}$alkyl; $R^7$ represents a hydrogen atom or a halogen atom or a group selected from $C_{1-6}$alkyl, $C_{2-6}$alkenyl or fluoro$C_{1-6}$alkyl; $R^8$ represents a hydrogen atom or a $C_{1-6}$alkyl group; $R^9$ represents a hydrogen atom or a group selected from phenyl or $C_{1-6}$alkyl, optionally substituted by a phenyl, hydroxyphenyl, hydroxyl, amino, guanidino, imidazolyl, indolyl, mercapto, $C_{1-6}$alkylthio, carboxyl or amido group; or $R^8$ and $R^9$, when taken together form an alkylene bridge of 2, 3 or 4 carbon atoms; $R^{10}$ represents a hydrogen atom or a $C_{1-6}$alkyl group; $R^{11}$ represents a group selected from hydroxyl, $C_{1-6}$alkoxy, $C_{2-6}$alkenoxy or $-NR^{12}R^{13}$; $R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom; m represents zero or an integer from 1 to 4; and n represents zero or an integer from 1 to 3; with the proviso that when n represents an integer from 1 to 3, $R^9$ and $R^{10}$ both represent hydrogen atoms, followed, if necessary, by removal of any protecting group present; or
(B) deprotecting a protected intermediate of general formula (IV)

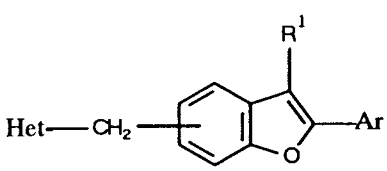

(IV)

in which at least one reactive group is blocked by a protecting group; or
(C) reacting a compound of general formula (Ia)

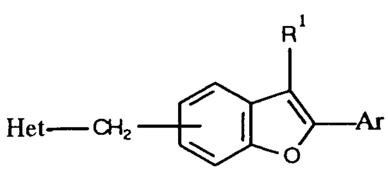

(Ia)

in which $R^3$ in the group Ar represents a nitrile group, with an azide, followed, if necessary, by the removal of any protecting group present; or
(D) reacting a compound of general formula (Ib)

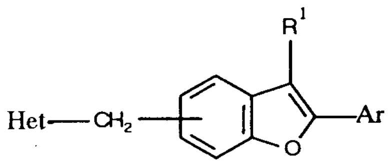

(Ib)

in which $R^3$ in the group Ar represents an amino group, with trifluoromethanesulphonic anhydride or trifluoromethylsulphonyl chloride, followed, if necessary, by removal of any protecting group present; or

(E) reacting a compound of formula (Ic)

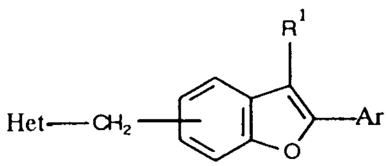

(1c)

in which Het represents the group

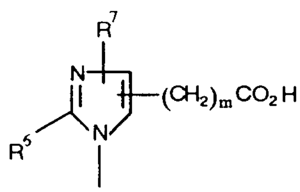

with a compound of general formula (V)

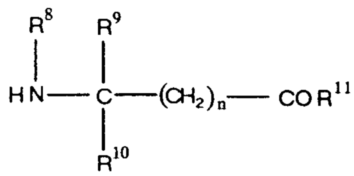

(V)

followed, if necessary, by removal of any protecting group present; and when the compound of general formula (I) is obtained as a mixture of enantiomers optionally resolving the mixture to obtain the desired enantiomer;

and/or, if desired, converting the resulting compound of general formula (I) or a salt thereof into a physiologically acceptable salt, solvate or metabolically labile ester thereof.

**19.** A pharmaceutical composition comprising at least one compound of general formula (I) as defined in any one of Claims 1 to 17 or a physiologically acceptable salt, solvate or metabolically labile ester thereof, together with at least one physiologically acceptable carrier or excipient.

**20.** A compound of general formula (I) as claimed in any one of Claims 1 to 17 or a physiologically acceptable salt, solvate or metabolically labile ester thereof for use in therapy, for example,

(i) for use in the treatment or prophylaxis of hypertension; or

(ii) for use in the treatment or prophylaxis of congestive heart failure, acute or chronic heart failure, aortic or cardiac insufficiency, post-myocardial infarction, renal insufficiency and renal failure (for example, as a result of diabetic nephropathy, glomerular nephritis, scleroderma or renal crisis), proteinuria, Bartter's syndrome, secondary hyperaldosteronism, Reynaud's syndrome, cerebrovascular insufficiency, peripheral vascular disease, diabetic retinopathy, atherogenesis and for the improvement of vascular compliance; or

(iii) for use in the treatment or prophylaxis of cognitive disorders such as dementia (e.g. Alzheimer's disease) and other CNS disorders, such as anxiety disorders, schizophrenia, depression and alcohol or drug (e.g. cocaine) dependency;
(iv) for use in the treatment of conditions associated with excessive or unregulated angiotensin II activity; or
(v) for use in the treatment of a condition associated with activation of the Renin-Angiotensin System.

**21.** A compound of general formula (Ia)

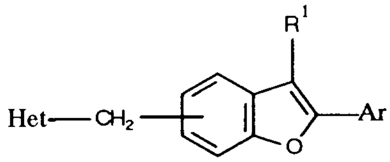

(Ia)

or an acid addition salt thereof
wherein
$R^1$, Ar and Het are as defined in Claim 1 except that in the group Ar, $R^3$ represents a nitrile group.

**22.** A compound of general formula (Ib)

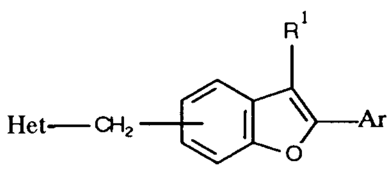

(Ib)

or an acid addition salt thereof
wherein
$R^1$, Ar and Het are as defined in Claim 1 except that in the group Ar, $R^3$ represents an amino group.

European Patent Office

# EUROPEAN SEARCH REPORT

Application Number

EP 92 10 4951

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-9 100 281 (E.I. DU PONT DE NEMOURS AND COMPANY)<br>* page 5, line 33 - page 20, line 2; claims 1,11 *<br>* abstract *<br>--- | 1,18,20 | C07K5/06<br>C07D405/14<br>C07D405/06<br>A61K31/41 |
| P,D,<br>A | EP-A-0 434 249 (GLAXO GROUP LIMITED)<br>* abstract; claims *<br>----- | 1,18,20 | |
| | | | TECHNICAL FIELDS SEARCHED (Int. Cl.5) |
| | | | C07K<br>C07D |

The present search report has been drawn up for all claims

| Place of search | Date of completion of the search | Examiner |
|---|---|---|
| THE HAGUE | 12 JUNE 1992 | PAISDOR B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document